(19) **Europäisches Patentamt / European Patent Office / Office européen des brevets**

(11) **EP 3 850 349 B1**

(12) **EUROPEAN PATENT SPECIFICATION**

(45) Date of publication and mention of the grant of the patent:
**20.03.2024 Bulletin 2024/12**

(21) Application number: **19860993.5**

(22) Date of filing: **30.08.2019**

(51) International Patent Classification (IPC):
*G01N 27/02* (2006.01)     *G01N 33/28* (2006.01)
*G01F 1/66* (2022.01)      *G01F 1/712* (2006.01)
*G01F 1/74* (2006.01)

(52) Cooperative Patent Classification (CPC):
**G01N 27/026; G01F 1/66; G01F 1/712; G01F 1/74; G01N 33/2823**

(86) International application number:
**PCT/US2019/049177**

(87) International publication number:
**WO 2020/055614 (19.03.2020 Gazette 2020/12)**

(54) **METHOD FOR IDENTIFYING AND CHARACTERIZING A CONDENSATE ENTRAINED WITHIN A FLUID**

VERFAHREN ZUR IDENTIFIZIERUNG UND CHARAKTERISIERUNG EINES IN EINEM FLUID MITGEFÜHRTEN KONDENSATS

PROCÉDÉ D'IDENTIFICATION ET DE CARACTÉRISATION D'UN CONDENSAT ENTRAÎNÉ DANS UN FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2018 US 201816131482**

(43) Date of publication of application:
**21.07.2021 Bulletin 2021/29**

(73) Proprietor: **Mohr And Associates, A Sole Proprietorship**
**Richland, WA 99354 (US)**

(72) Inventors:
• **MOHR, Charles L.**
  **Richland, WA 99352 (US)**
• **MOHR, Brandt C.**
  **Richland, WA 99352 (US)**
• **COTTAM, Anthony**
  **Richland, WA 99354 (US)**
• **MAY, Preston**
  **Richland, WA 99352 (US)**
• **KENNY, Daniel**
  **Richland, WA 99352 (US)**
• **RAUSCH, William**
  **Richland, WA 99354 (US)**
• **HURLEY, David**
  **Pasco, WA 99301 (US)**
• **NGUYEN, Duan**
  **Richland, WA 99352 (US)**
• **DAWES, Kevin**
  **Richland, WA 99354 (US)**
• **VON REIS, Erik**
  **Kennewick, WA 99338 (US)**
• **MULKEY, Christopher**
  **West Richland, WA 99353 (US)**
• **SAMS, Ryan**
  **Kennewick, WA 99336 (US)**
• **MOHR, Benno**
  **Pullman, WA 99163 (US)**
• **STORDAHL, Michael**
  **Kennewick, WA 99338 (US)**

(74) Representative: **Schmid, Wolfgang**
**Lorenz & Kollegen**
**Patentanwälte Partnerschaftsgesellschaft mbB**
**Alte Ulmer Strasse 2**
**89522 Heidenheim (DE)**

(56) References cited:
**US-A- 5 586 085        US-A1- 2007 279 073**
**US-A1- 2014 085 133    US-A1- 2017 350 801**
**US-A1- 2017 350 842    US-A1- 2017 350 843**

**Description**

**TECHNICAL FIELD**

This invention relates to a method for identifying, characterizing and measuring condensates entrained in a liquid, and more particularly to a method for identifying, characterizing and measuring volume fractions of "wet gases" entrained within transient "slugs" of liquid periodically released into, and passing through natural gas wells, and related production apparatus, using reflected electrical signals and resonance points.

**BACKGROUND OF THE INVENTION**

US2017/350843 A1 refers to a probe for identifying and measuring volume fraction constituents of a fluid using time domain analysis and frequency domain analysis to identify individual volume fraction constituents within a pipe on a real time basis and to measure the volume of the individual volume fraction constituents flowing through the pipe on a real time basis.

Natural gas is a gaseous mixture comprised of multiple different hydrocarbons and can exist in a gaseous phase as well as liquid phase depending upon temperature and pressure. The most prevalent hydrocarbon within natural gas is methane. The higher the methane concentration, the "drier" or "colder" the natural gas is considered. Other hydrocarbon constituents of natural gas are evaporated liquids such as, but not limited to, ethane, butane, pentane, propane and hexane. These other hydrocarbon constituents are collectively referred to as "condensates" or Natural Gas Liquids (NGL's). The higher the concentration of condensates, or NGLs, the "wetter" or "hotter" the natural gas is considered.

"Condensates" and "NGLs" have significantly higher economic value than methane, and thus "wet gases" are worth considerably more than "dry gases". The market price for condensates, and NGLs, can be many multiples of the market price for methane. Therefore, an accurate assessment and accounting of the volumes of such condensates and NGLs within natural gas is to the economic benefit of well owners and operators.

"Condensates" or "NGL's" must be separated/removed from the natural gas and methane, before the natural gas can be placed in a pipeline and sold/consumed by the general public.

The current practice in the petroleum/fuel industry for identifying measuring the constituents of natural gas and other components being produced by a given natural gas well, or group of natural gas wells, is to separate the produced components in a three phase separator and to identify and measure the produced components individually. Three phase separators are typically large, expensive, maintenance intensive and typically provide production information only after long intervals during which the hydrocarbon components, and other products, separate under the influence of gravity. Furthermore, such three phase separators are generally not operated continuously, but are rather used only during testing and certification of the wells. As such, such three phase separators are not available for periodic and transient releases of wet gases.

The "condensates" are not continuously, evenly, or regularly released into a natural gas well. Instead, such condensates are more commonly periodically and transiently released into a natural gas well as a liquid "slug" wherein the condensates are entrained in water. The occurrence and release of such "slugs" is not predictable, nor regular. Depending upon individual wells, and the geological formations in which the wells are drilled, such slugs may be released/occur as often as multiple times per hour, or as infrequently as only once or twice per several days. Because the "slugs" are primarily liquid, they are highly concentrated volumes of the condensates. (The condensates are in a liquid phase as opposed to a gaseous phase).

The transient and periodic nature of these slug releases exacerbates the problem of identifying and measuring the various condensates comprising the slugs. Three phase separators that are used to provide well certifications, are typically not operated continuously, but rather are only operated periodically during the well certification. The periodic operation of the three phase separators, combined with the periodic and transitory nature of the slug releases has made the accurate and consistent measurement and identification of condensates nearly impossible.

Due to the significantly enhanced economic value of the condensates, as opposed to the methane, it is desirable to be able to accurately identify, characterize and measure the condensates that are periodically and transiently released into natural gas wells so that owners and operators of such wells can be accurately and fairly compensated for the specific hydrocarbons produced by the well.

The instant inventive method herein provides a method for addressing the identification, characterization and measurement of the transient periodic release of the condensates. A typical periodic and transient release of liquid containing condensates is approximately 200 seconds in duration and the release contains water as well as various liquid and gaseous hydrocarbon components that make up the primary wet gas composition. The volume fractions are at a maximum concentration with the least amount of dilution by methane gas bubbles at the beginning of the transient release. (the slug). The beginning of the transient release provides the most opportune time to make a measurement of the condensate composition. The measurement opportunity is at its best at the beginning of the transient release and will last for a period of approximately two (2) seconds to about seven (7) seconds, calculated from the beginning of the release. The measurement will remain functional throughout the periodic transient release, but the best time to characterize and measure the liquid/condensate composition will be at the beginning of the release. The volume of the release can be measured by the characteristic cross correlation between a first measurement probe and a second measurement probe.

The water/wet gas concentration begins tapering downwardly from the beginning of the release with an increasing amount of methane gas bubbles within the condensate. As the pressure drops, some of the wet gas components will change into the gas phase as the initial liquid slug is dissipated.

According to the invention, the best opportunity for the measurement of the liquid hydrocarbon, versus the water fraction using electric field perturbation, based on time domain reflectometry methods, occurs close to the leading edge of the liquid slug.

Two EFP measurement probes are fixed within a bore of the pipe/conduit a set distance from one another. System pressure and system temperature sensors are also mounted to, and communicate with the fluid within the bore of the pipe/conduit. The sensors are exposed to the fluid flow. The multiphase electronics sampling occurs at approximately 500 frames per second at each of the two EFP probe locations and provides data to characterize the constitutes at each probe station.

Using cross correlation methods, the velocity of the mixture flow within the conduit/pipe can be calculated. Using the average composition calculated from the two EFP measurement probes, the total flow volume and the fractional volume of the hydrocarbon-based constituents and condensates can be estimated. As a result, the total flow and volume of the hydrocarbon-based constituents and condensates can be estimated.

As a result of the slug flow analysis, the characterization of the fractional constituents of the flow, in conjunction with system pressure and temperature, permit an approximation of the fluid density at any point in time. This fluid density measurement may be used in conjunction with an optional external flow measurement device to provide another estimate of the total volume flow of the material through the pipe, which can then be used to improve the resultant calculation of each fluid constituent.

To address the drawbacks of three-phase separators, composition meters have been developed. When a composition meter is combined with a flow meter, production rates for the various components may also be roughly estimated. Composition meters use measurement of dielectric constant, in combination with a density measurement, to determine the volume fractions.

For known composition meters to be consistently accurate, all the dielectric constants and all the densities of the individual produced fluid components must be known for every measurement condition (temperature and pressure). Unfortunately, this is nearly impossible to accomplish because the conditions are continually varying and changing as the well, or group of wells, produce. Accuracy of the measurements is further complicated by several of the lower density hydrocarbon components (for example but not limited to, ethane, propane, butane and pentane) existing in either a liquid state or a gaseous state at pressures between approximately 20 and 250 atmospheres. Further, the produced components are typically at very high temperatures and as a result, produced water boils off into steam within the pipes causing identification and measurements of gaseous components to be particularly difficult because the dielectric constant of steam is very close to the dielectric constants of the lower density hydrocarbon components.

Prior art publications claim it is "impossible" to accurately identify and measure the volume fractions of natural gas without knowing how much of each hydrocarbon constituent is in the liquid or gaseous phase at any given time.

A further complicating factor in measuring volume fraction constituents of mixtures of produced natural gas is the salt content of the mixture. The salt also affects the dielectric constant of the fluid components.

Our method for identifying and characterizing a condensate entrained within a fluid overcomes various of the drawbacks of known methods and apparatus.

## SUMMARY OF THE INVENTION

The present invention provides a method for identifying, and characterizing a periodic release of a given condensate which is entrained within a source of a fluid according to independent claim 1.

A further aspect of the present invention is a method that further comprises providing an elongated conduit or pipe having an internal bore which has a predetermined, substantially uniform, inside diametral dimension; providing two electric field perturbation probes, and positioning each of the electric field perturbation probes, at least in part, within the bore of the elongated conduit, and at a known, and predetermined distance, one relative to the other; providing a fluid pressure sensor which is mounted in a fluid pressure sensing relationship relative to the internal bore of the elongated conduit, and which further generates a fluid pressure signal; providing a temperature sensor which is mounted in temperature sensing relation relative to the internal bore of the elongated conduit, and which further generates fluid temperature signal; electrically coupling the two electric field perturbation probes, fluid pressure sensor, and temperature sensor in a signal transmitting relationship relative to the computer; delivering the source of the fluid which includes the major volume fraction constituent, and the at least one condensate, into the internal bore of the elongated conduit; electrically sampling, with the computer, each of the respective two electric field perturbation probes, fluid pressure, and/or temperature sensor signals; and correlating the signals received from the at least two electric field perturbation probes, the pressure sensor and/or temperature sensor, with the computer, so as to provide a characterization of the source of the fluid.

A further aspect of the present invention is a method wherein each of the electric perturbation sensors are located at predetermined, spaced apart, sensing stations which are located along the elongated conduit, and electrically sampling with the computer, at each of the sensing stations, at a speed of about 500 frames per second.

Another aspect of the present invention is a method that further comprises calculating, with the computer, a flow velocity of the source of the fluid through the internal bore of the elongated conduit from the signals received from the two electric field perturbation probes, and the temperature and fluid pressure sensors.

A further of the present invention is a method that further comprises characterizing, with the computer, the average composition of the source of the fluid in the region of the respective, spaced, sensing stations by utilizing the signals received from the respective, electric field perturbation sensors; and estimating, by utilizing the computer, a total flow volume of the source of the fluid, and a fractional volume of the at least one hydrocarbon condensate which is entrained with the source of the fluid.

A further aspect of the present invention is a method that further comprises calculating, with the computer, an approximate fluid density of the source of the fluid, by utilizing the signals received from the temperature and pressure sensors, during a given sampling time; and providing an auxiliary, and externally mounted fluid flow measurement device and coupling the auxiliary, and externally mounted fluid flow measurement device in a signal transmitting relationship relative to the computer; delivering the source of fluid to the auxiliary, and externally mounted, fluid flow measurement device, and generating a signal with the auxiliary and externally mounted fluid flow measurement device which is transmitted to the computer; measuring the fluid flowing movement of the source of the fluid through the auxiliary, and externally mounted, fluid flow measurement device; estimating the total flow of the source of fluid, with the computer, by utilizing the signal generated by the auxiliary, and externally mounted, fluid flow measurement device; and improving the estimated calculation of the total flow volume of the source of the fluid, and the fractional volume of the at least one hydrocarbon condensate which is entrained with the source of the fluid, by utilizing the estimated total flow of the source of fluid, and which is detected by the auxiliary, and externally mounted fluid flow measuring device, by employing the computer.

Another aspect of the present invention is a method that further comprises applying a Fourier Transform calculation to the sensed electrical pulse reflection received from the probe, and which is used to determine a resonant frequency and resonance point of at least one of the volume fraction constituents.

A further aspect of the present invention is a method that further comprises applying a Power Spectral Density (PSD) calculation, by means of the computer, to the Fourier Transform (FFT) frequency so as to determine an amplitude, and strength of at least one of the given resonance points.

Another aspect of the present invention is a method wherein the volume fraction constituent is a multiplicity of volume fraction constituents.

A further aspect of the present invention is a method wherein the volume fraction constituents includes a liquid and a gas.

## BRIEF DESCRIPTIONS OF THE DRAWINGS

Figure 1 is a generalized block diagram of our apparatus showing arrangement of the various components and fluid flow therethrough.

Figure 2 is an orthographic front view of the two representative spaced apart grayloc supports and an electronics box mounted on a moveable support skid.

Figure 3 is an exploded isometric front, side and top view of a grayloc support showing arrangement of the components and the probe.

Figure 4 is an orthographic side view of the assembled grayloc support of Figure 3, less the sealed hubs.

Figure 5 is an orthographic cross section view of the assembled grayloc support of Figure 4 taken on line 5-5 from Figure 4.

Figure 6 is an isometric front, side and top view of a first configuration of a probe and support block.

Figure 6A is an enlarged isometric view of the probe and support block showing details of the coaxial cable connection.

Figure 7 is an exploded isometric front, side and top view of the probe of Figure 6.

Figure 8 is an orthographic front view of the probe of Figure 6 less the support block.

Figure 9 is an isometric front, side and top view of a second configuration of probe having offset ground plates.

Figure 10 is an orthographic side view of the second configuration of blade probe of Figure 9, showing the open structure formed by offsets of the ground plates relative to the center conductor.

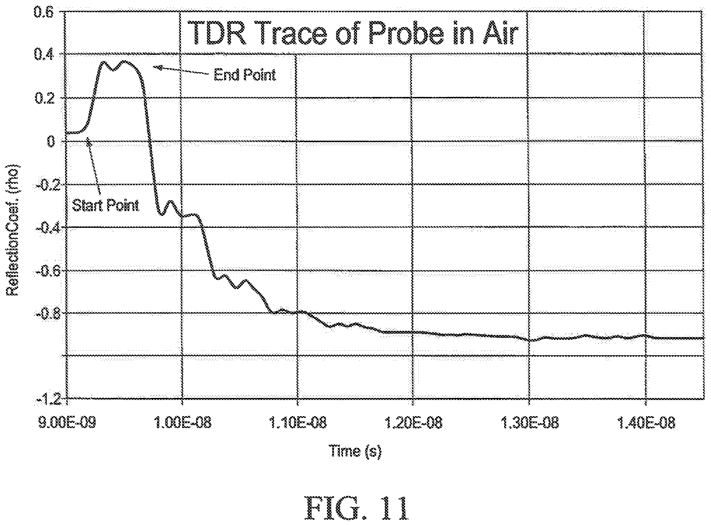

Figure 11 is a time domain reflectance trace of an electrical pulse through the probe in air showing the start point and the end point.

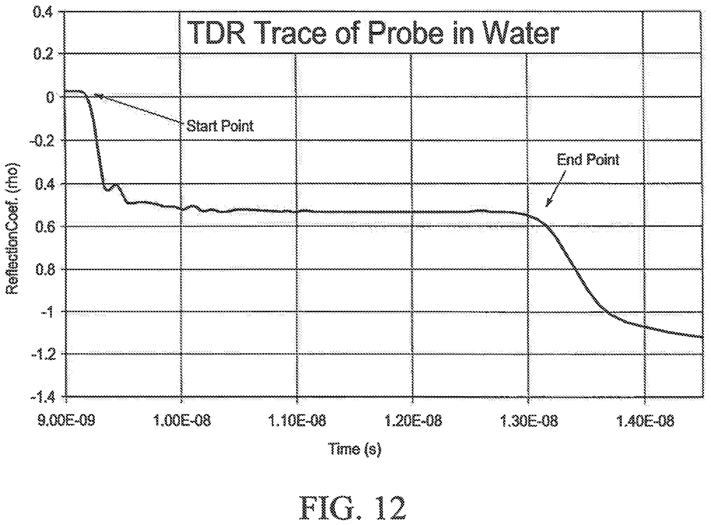

Figure 12 is a time domain reflectance trace of an electrical pulse through the probe in water showing of the start point and the end point.

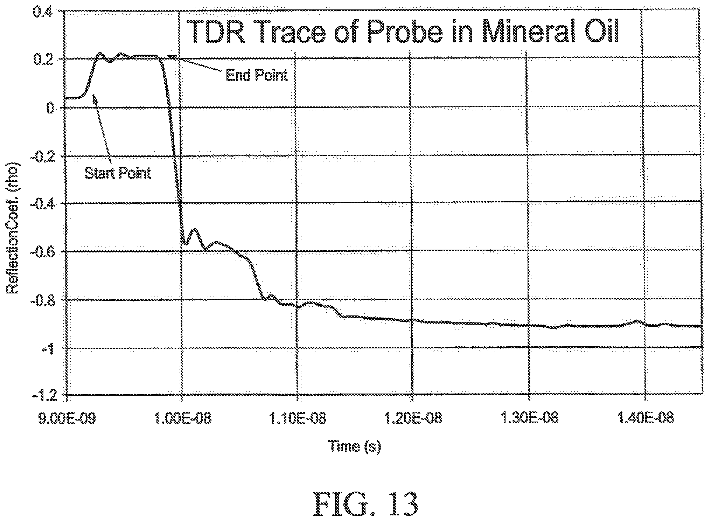

Figure 13 is a time domain reflectance trace of an electrical pulse through the probe in mineral oil showing the start point and the end point.

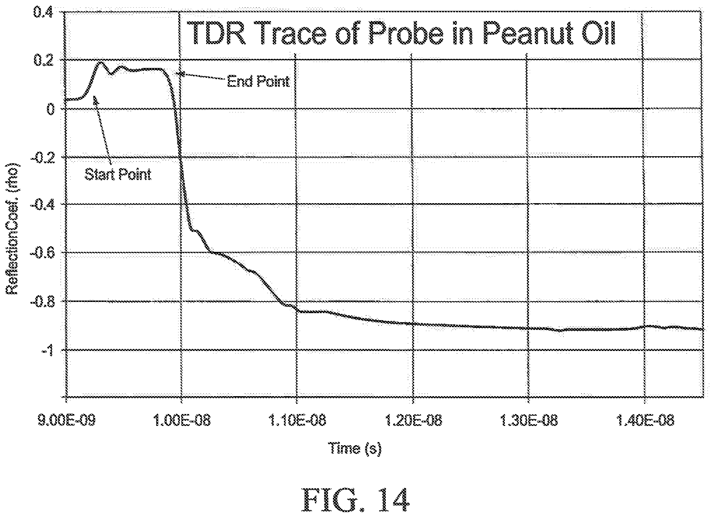

Figure 14 is a time domain reflectance trace of an electrical pulse through the probe in peanut oil showing the start point and the end point.

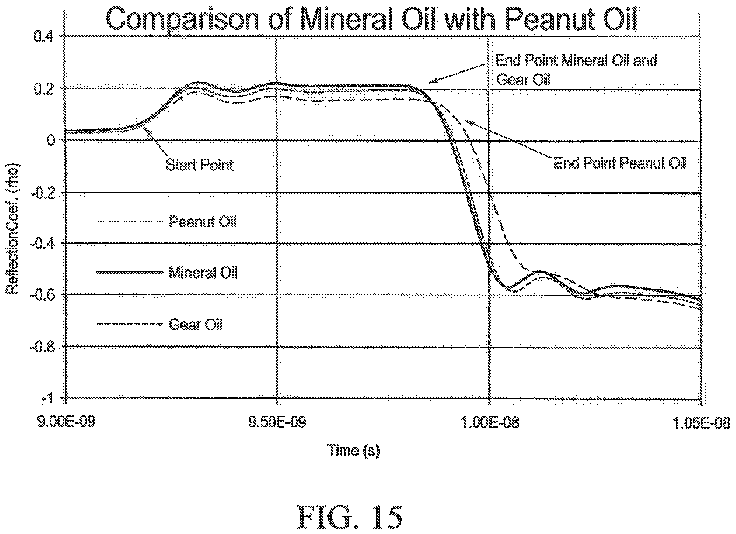

Figure 15 is a comparison time domain reflectance trace of an electrical pulse through the probe in peanut oil, mineral oil and gear oil showing the start point and the endpoint and showing the similarity in the traces amongst the different types of oils.

Figure 16 is a time domain reflectance trace of an electrical pulse through the probe in a mixture of air, mineral oil, peanut oil and water showing the differences in the traces which allows identification of the components.

Figure 17 is a power spectral domain (frequency domain evaluation) graph of the TDR traces of Figure 16 after applying the FFT and PSD showing the resonance points of the components.

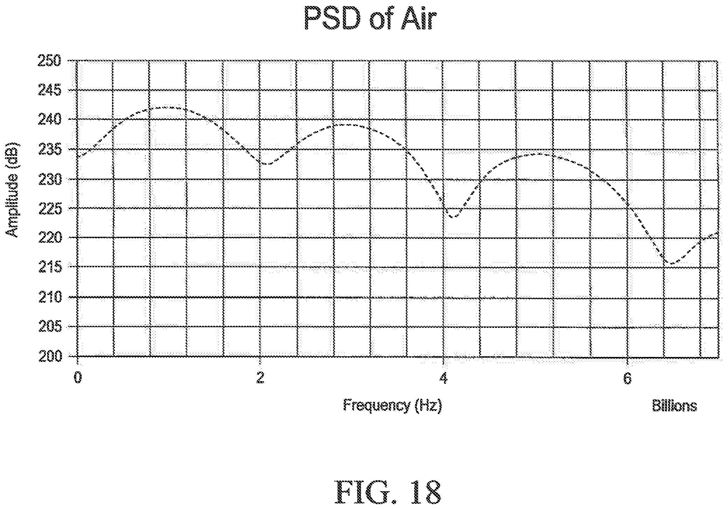

Figure 18 is a power spectral domain (frequency domain evaluation) graph of the TDR trace of Figure 11 showing the resonance points in air.

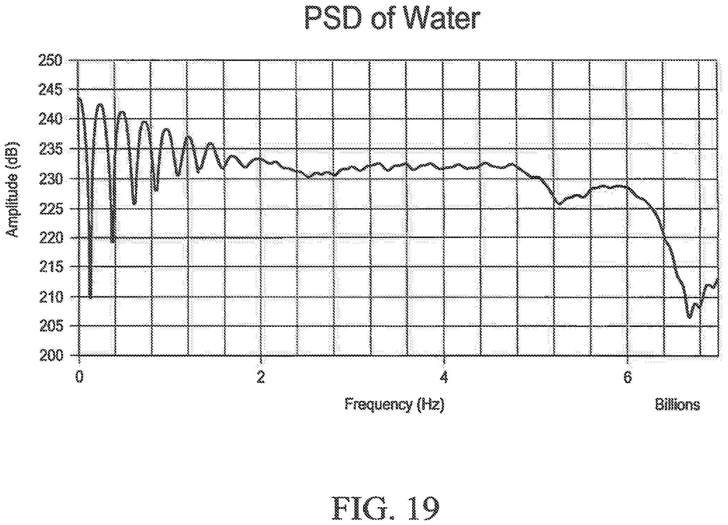

Figure 19 is a power spectral domain (frequency domain evaluation) graph of the TDR trace of Figure 12 showing the resonance points in water.

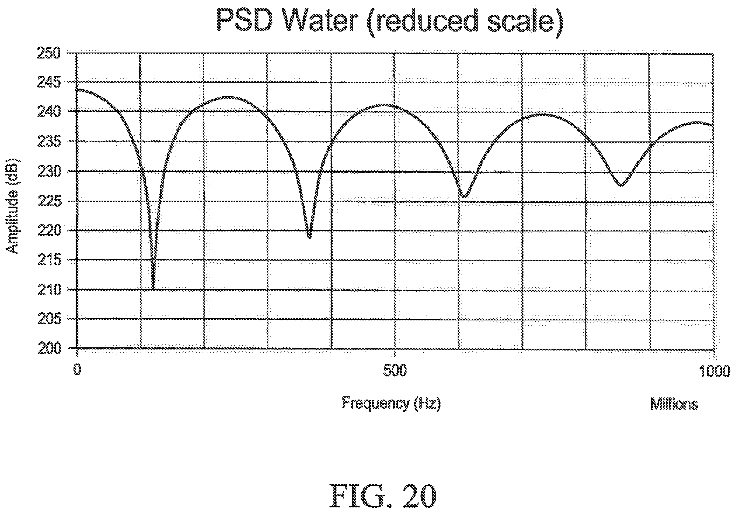

Figure 20 is a reduced scale power spectral domain (frequency domain evaluation) of the probe in water, similar to that of Figure 19 showing the resonance points.

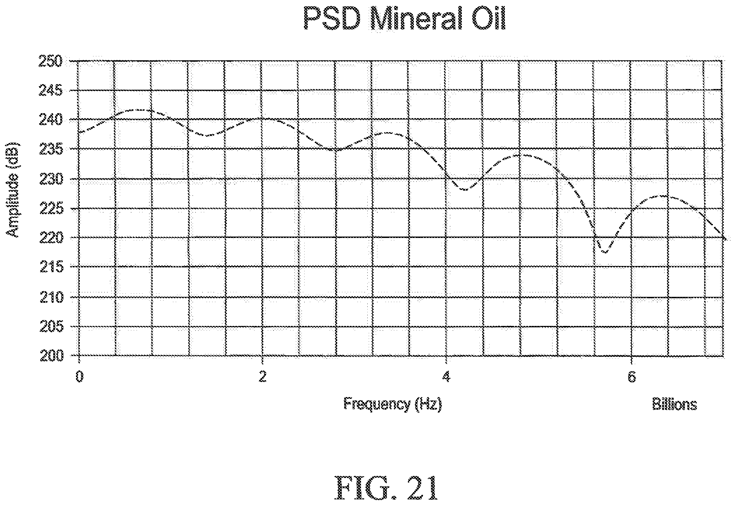

Figure 21 is a power spectral domain (frequency domain evaluation) graph of the TDR trace of Figure 13 showing the resonance points in mineral oil

Figure 22 is an artistic representation of a periodic transient slug of liquid comprised of a variety of condensates passing through a conduit/pipe showing the direction of flow, the leading edge of the slug and the trailing edge of the slug.

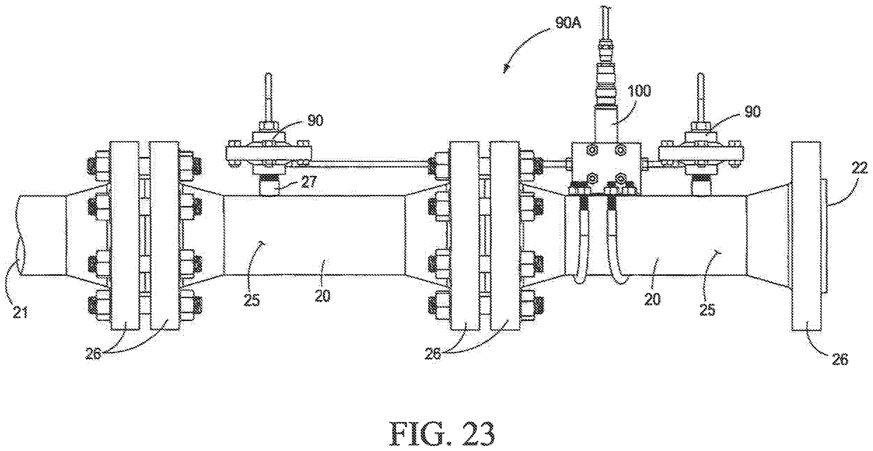

Figure 23 is an orthographic front to view of a representative optional additional flow meter assembly that may be utilized in the instant inventive method.

Figure 24 is an orthographic front view of a third embodiment of an EFP probe, known as a "through" probe.

Figure 25 is an orthographic first side view of the third embodiment of EFP probe of Figure 24.

## DETAILED DESCRIPTION OF THE PREFERRED EMBODIMENTS

A method for identifying and characterizing a condensate entrained in a fluid generally comprises a source of fluid 13, a conduit or pipe 20, a probe 30, a grayloc support 80, a pulse emitter 120, a pulse sampler 150, a computer 170, a support frame 200, and optionally an externally mounted fluid flow measurement device 90A.

The source of fluid 13 is typically a producing natural gas well, or grouping of natural gas wells producing a fluid 14 that contains a mixture of various volume fractions including, but not limited to, oil 15, water 16, and various forms of hydrocarbon natural gas 17 including, but not limited to, ethane 17A, butane 17B, pentane 17C and propane 17D, and condensate 19. The various hydrocarbon condensates 19 including, but not limited to ethane 17A, butane 17B, pentane 17C and propane

17D may, at least partially comprise, be entrained in the condensate 19, and may also be in the form of an emulsion 18. When produced from the source of fluid 13, the fluid 14 is at pressure and is typically at a temperature that may exceed ambient temperature by hundreds of degrees, although the temperature and pressure vary over time and conditions. It is further contemplated and anticipated the fluid 14 volume fraction constituents 15, 16, 17, 17A, 17B, 17C, 17D, 19 may be produced, and flow through the pipe 20, in segregated fashion, and at other times it is anticipated the volume fraction constituents 15, 16, 17 will be a mixture or emulsions 18 of fluid 14 that may or may not be homogeneously distributed within the pipe 20.

Oil 15, water 16 natural gas 17 and condensate 19 are different molecular compounds, and have different, well recognized dielectric constants and resonance points depending upon the concentration. The dielectric constant of water 16 ranges from approximately 80 for cold water down to approximately 25 for very hot water. The dielectric constant of steam is approximately 1.01 increasing to approximately 1.15 as temperature increases. The dielectric constant of oil 15 is approximately 2.0 to 2.5 depending upon the density of the oil 15. The dielectric constant of natural gas 17 and the various hydrocarbons making up the condensate 19 is approximately 1.2 to approximately 1.8.

Because the known dielectric constant of steam (approximately 1.01-1.15) is similar to the dielectric constant of natural gas 17 and condensate 19 (approximately 1.2-1.8) use of a back pressure regulator 110 communicating with the pipe 20 maintains pressure within the pipe 20 at a pressure at least equal to the pressure of the fluid 14 exiting the source of fluid 13. With the use of a back pressure regulator 110, even though the fluid 14 may be at an extremely high temperature, the water 16 within the fluid 14 will not boil, and will remain in a liquid state with the corresponding dielectric constant and resonance points which are measurably different than the dielectric constant of natural gas 17 and condensate 19. Preventing the formation of steam inside the pipe 20 allows the instant apparatus to distinguish between natural gas 17 and condensate 19, and water 16 using the known dielectric constants and resonance points thereof.

The conduit or pipe 20 has an inflow end 21 communicating with the source of fluid 13 and an outflow end 22 communicating with a distribution point (not shown) such as a collection facility (not shown). The pipe 20 has a known interior diameter 23, an exterior diameter 24, an exterior surface 25, defines a medial channel 28 and may contain a plurality of connections 26 where fittings 27 and apparatus and the like may be joined to the pipe 20, and also where the pipe 20 may connect to other sections of pipe 20 to extend the length thereof.

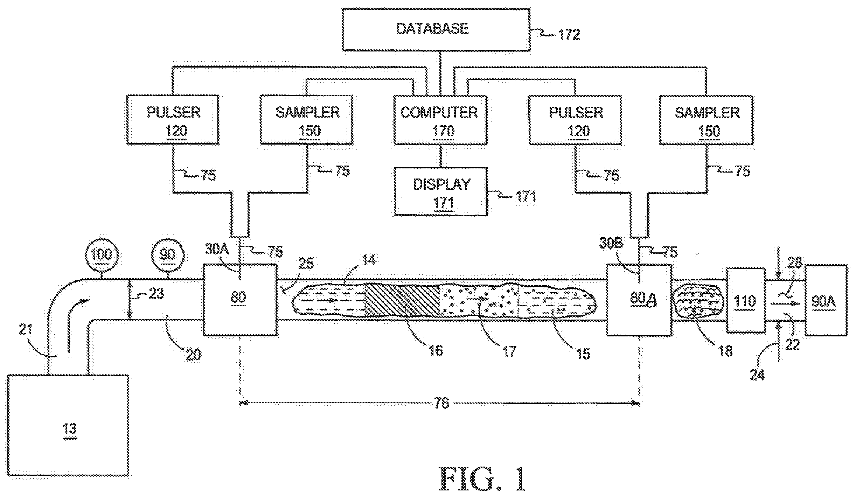

As shown in Figure 1, a temperature sensor 100 and a flow meter 90 may be interconnected with the pipe 20 downstream of the source of fluid 13 and upstream of the grayloc support 80. The temperature sensor 100

and flow meter **90** are known apparatus and communicate with the medial channel **28** of the pipe **20** to monitor and sense the temperature of and movement of fluid **14** through the pipe **20**. Information and data sensed by the temperature sensor **100** and the flow meter **90** are communicated to the computer **170**.

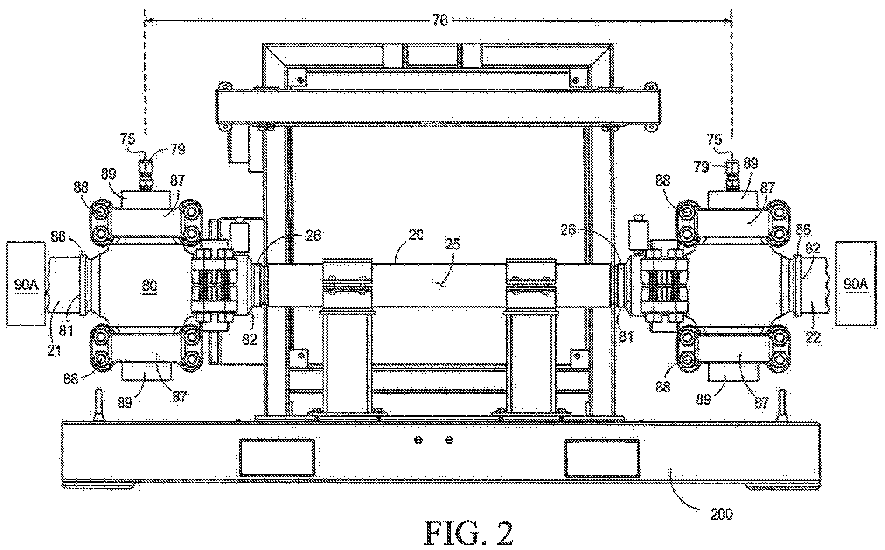

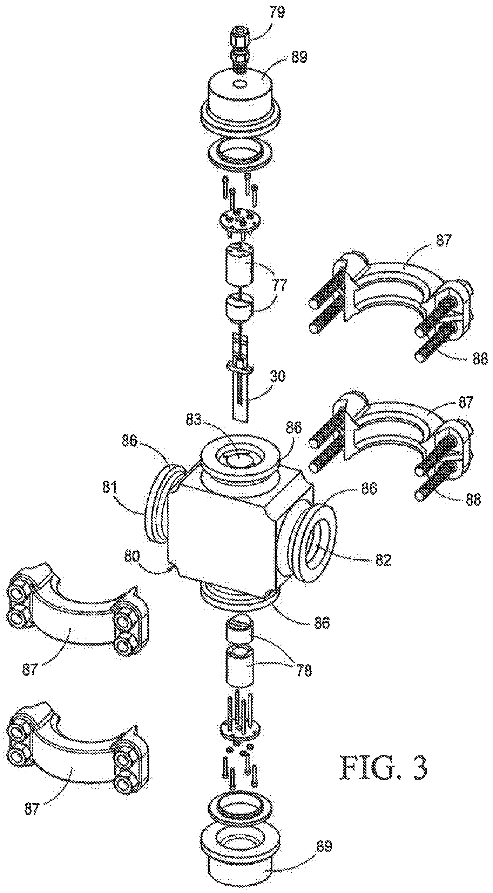

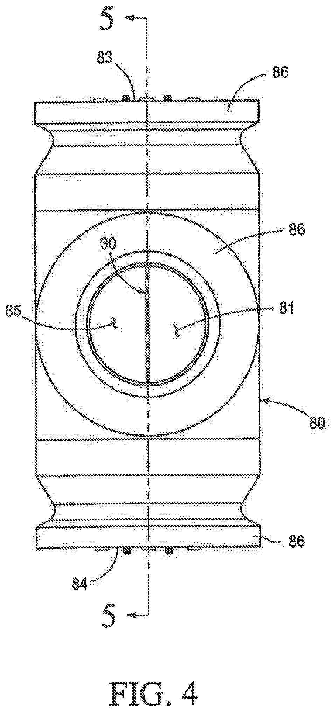

In a first embodiment of the invention (Figure 2), there are two spaced apart grayloc supports **80**, **80A**. Each grayloc support **80**, **80A** (Figures 3-5) is a fitting having a "cross" configuration defining an entry port **81**, an exit port **82**, a probe insertion port **83** and a blind port **84**. Each of the ports **81**, **82**, **83**, **84** communicate with a medial chamber **85** therebetween to allow fluid **14** flow therethrough. An exterior circumference of each port **81**, **82**, **83**, **84** defines a radially enlarged sealing flange **86** configured for engagement with a sealing clamp **87** to provide a fluid tight seal between the grayloc support **80** and the adjoining pipe **20**, or an adjoining hub **89** to provide fluid containment.

As shown in Figure 2, the second grayloc support **80A** communicates with the pipe **20** a known distance **76** downstream from the first grayloc support **80**. The second grayloc support **80A** has the same components and configuration as the first grayloc support **80** and therefore a detailed description of the second grayloc support **80A** is omitted herein.

In the first embodiment there are two spaced apart probes **30A**, **30B**, one probe **30** within each grayloc support **80**, **80A**. The first probe **30A** and the second probe **30B** are identical in configuration, and in function, and therefore only the first probe **30A** will be described in detail. These two spaced apart grayloc supports **80** allows velocity and volume to be calculated without use of a flow meter **90**, although an external and optional flow meter assembly **90A** may be interconnected with the conduit or pipe **20** to provide additional information to the computer **170** to enhance the accuracy of the measurement data, and to provide a separate and independent data point for cross-correlation employing the instant inventive method.

As shown in Figures 3, 4 and 5, the probe **30** is positionally supported within the medial chamber **85** defined by the grayloc support **80** so that at least a portion of the probe **30** is exposed to the fluid **14** flowing through the grayloc support **80** medial chamber **85**.

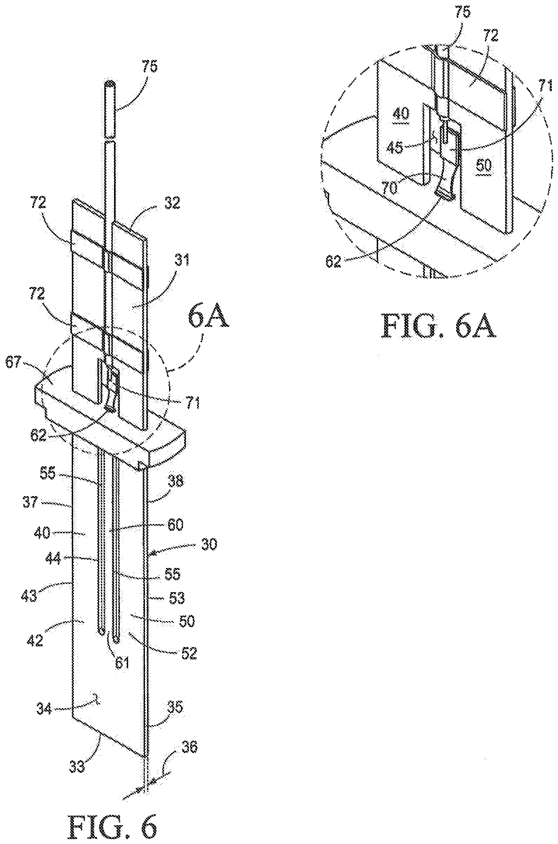

The probe **30** (Figures 6-8) has a body **31** that is generally planar and rectilinear. The body **31** has a first end **32** and an opposing second end **33**, a first surface **34**, and an opposing second surface **35** with a thickness **36** between the first surface **34** and the second surface **35**. The body **31** further has a first laterally outer edge **37**, and an opposing second laterally outer edge **38** and defines a dimensionally enlarged shoulder (not shown) in the first edge **37** and the second edge **38** spaced apart from the first end **32** to positionally support to a probe support block **67**. The body **31** further defines an elongated medial slot **45** between a first ground plate **40** at the first edge **37** and a second ground plate **50** at the second edge **38**. An elongated center conductor **60** is carried within the medial slot **45** and has a root end **61** that is structurally attached to the probe body **31** proximate the second end **33** between the first and second ground plates **40**, **50** respectively, and the center conductor **60** has a free terminal end **62** within the medial slot **45** proximate to the body **31** first end **32**. The free terminal end **62** of the center conductor **60** carries a conductor adaptor link **70** and a conductor weld pad **71** for electronic connection to a coaxial cable **75**. The length **66** of the center conductor **60** defines the active length **66** of the probe **30**. The first end **32** of the probe body **31** is known as the "active end" of the probe **30**.

An elongated gap **55** is defined between each laterally outer edge of the center conductor **60** and a proximate edge of the first ground plate **40** and a proximate edge of the second ground plate **50**. The gap **55** is engineered to provide optimum sensitivity to the detection of charges in volume flow constituents **15**, **16**, **17**, **19** by impedance measurements. The gap **55** is uniform along its length and is typically approximately 2 mm (0.080 inches) in width for oil **15**, water **16** and natural gas **17** mixtures. It is expressly contemplated however, other gap **55** widths may be used and/or engineered to match the impedances of other volume fraction constituents **15**, **16**, **17**, **19** to be identified and measured in the fluid **14**.

A probe support block **67**, which is generally rectilinear in configuration and formed of silicon carbide defines a generally medial slot (not shown) therein through which the probe body **31** first end **32** extends. The probe support block **67** frictionally engages with the dimensionally enlarged shoulders (not shown) defined in the probe body **31** so as to positionally maintain the probe **30** relative to the probe support block **67**.

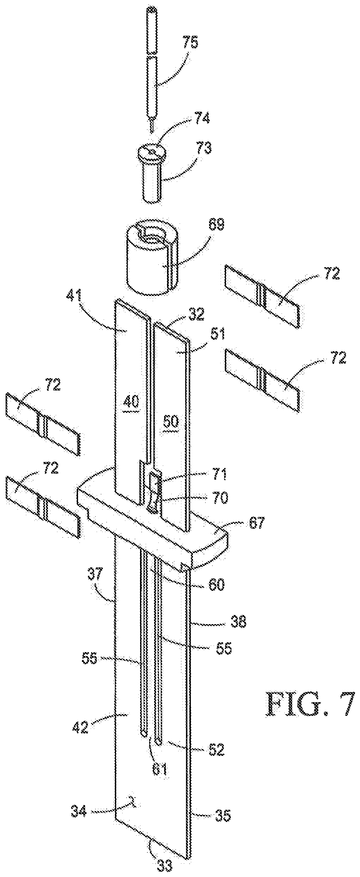

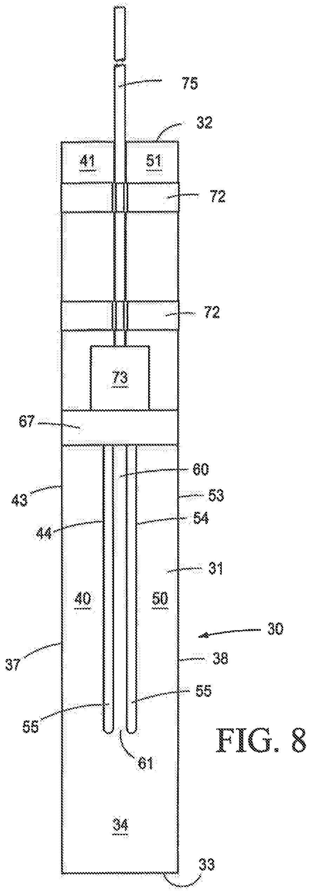

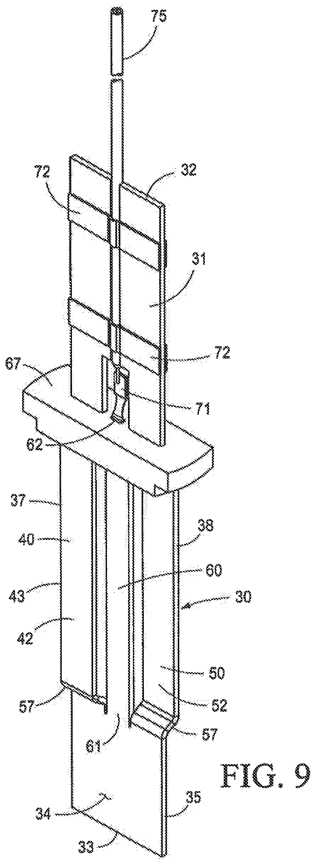

A coaxial cable 75 is electronically coupled with the conductor weld pad 71 so that signals may be transmitted to the probe 30 and received from the probe 30. Best shown in Figure 7, the coaxial cable 75, and its attachment to the conductor weld pad 71, is positionally secured to the probe body 31 by an inner slip support 69, a pack 73 and a ring 74 so that the coaxial cable 75 is securely, and insulatively connected to the center conductor 60. In the current embodiment the pack 73 and ring 74 are formed of TEFLON, but other materials such as PEEK may similarly be used and one contemplated. Plural support straps 72 (Figures 8, 9) spacedly arrayed on the probe body 31 further secure the coaxial cable 75 relative to the probe 30.

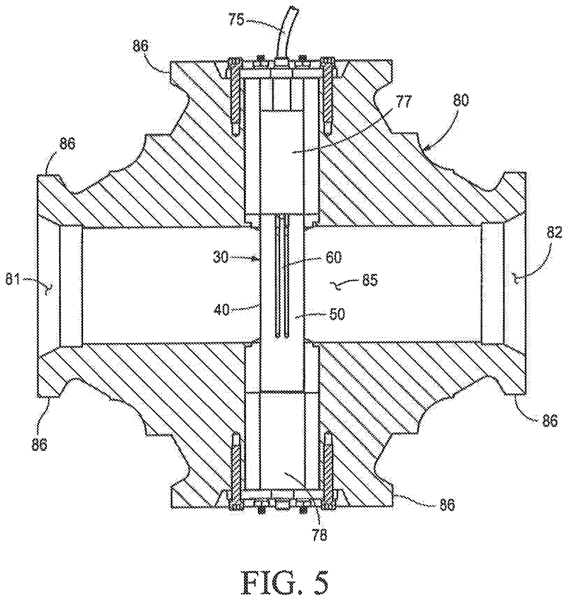

An active end support **77** (Figure 3) frictionally engages the first end **32** of the probe **30** and extends over and about the coaxial cable **75** and an inner slip support **69**. The active end support **77** aligns and positionally maintains the first end **32** of the probe body **31** within the medial chamber **85** of the grayloc support **80**. (See Figure 5). Similarly, a passive end support **78** frictionally engages with the second end **33** of the probe **30** and similarly aligns and positionally maintains the second end **33** of the probe **30** within the medial chamber **85** of the grayloc support **80**. (Figure 5).

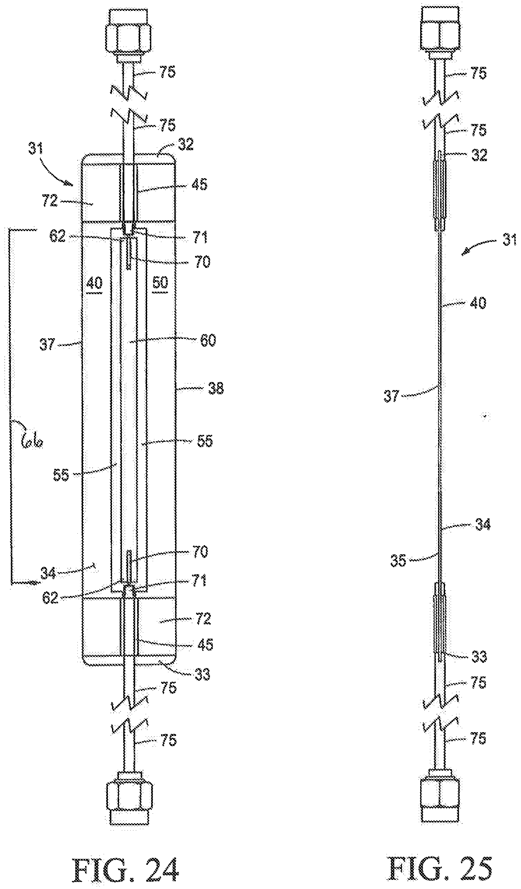

When the current design blade probe **30** is utilized the reflected signal (not shown) is electrically returned to the TDR via the coaxial cable **75** and is the sampled reflection. When a double ended or "through" probe 30 (Figures 24, 25) is utilized, the signals pass entirely through the active length **66** of the probe **30** from the first end **32** to the second end **33** and the signals are communicated to the pulse sampler **150**.

As shown in Figure 3, the assembled probe **30** and the active end support **77** are inserted into the grayloc support **80** probe insertion port **83** so that a medial portion of the probe **30** extends across the medial chamber **85** and is oriented so that the first surface **34** and second surface **35** are parallel to the flow of fluid **14** through the grayloc support **80** medial chamber **85**. The probe **30** and end supports **77**, **78** are secured within the grayloc support **80** medial chamber **85** by known means including, but not limited to, a spacer, a retainer plate and alignment pins. Such fastening means secure the first end **32** of the probe **30**, and also secure the second end **33** of the probe **30** so that the probe **30** is supported from both the first end **32** and the second end **33** within the medial chamber **85**. A fluid tight hub **89** is interconnected with the probe insertion port **83** sealing flange **86**, and also with the blind port **84** sealing flange 86. Known sealing clamps **87**, and plural threaded fasteners **88** secure the hubs **89** to the sealing flanges **86** to provide a fluid tight seal therebetween. As can be seen in the drawings, the coaxial cable **75** extends through the hub **89** proximate to the first end **32** of the probe **30** by way of a CONAX pressure gland seal **79**. The coaxial cable **75** electronically communicates with the probe **30** center conductor **60** and with the pulse emitter **120** and with the pulse sampler **150**.

The grayloc entry port **81** communicates with the pipe **20** by means of a fluid tight connection **26** therebetween. Similarly, the exit port **82** communicates with a pipe **20** by means of a fluid tight connection **26** therebetween.

The second grayloc support **80A** is also in fluid communication with the pipe **20** a known distance **76** downstream from the first grayloc support **80**. The structure of the second grayloc support **80A**, and the structure of the second probe **30B** carried therein is the same as the aforementioned and described grayloc support **80** and first probe **30A**.

The coaxial cables **75** that electronically communicate with each of the probes **30A**, **30B** are each electronically coupled with a pulse emitter **120** and also with pulse sampler **150**. The pulse emitter **120** and the pulse sampler **150** may also be combined into a single apparatus commonly called a Time Domain Reflectometer (TDR), such as the EFP Signal Processor utilizing the CT100B software developed and manufactured by Mohr Test and Measurement of Richland, Washington, USA. Such TDR EFP Signal Processors are described in U.S. patents US 4,786,857 issued November 22, 1998, and US 5,723,979 issued March 3, 1998, and US 6,144,211 issued November 7, 2000, and US 6,348,803 issued February 19, 2002 and which were all invented by Charles L. Mohr (one of the joint inventors herein).

Time domain reflectometry is an effective means for determining the level of a liquid, such as in a tank. Using time domain reflectometry, electrical pulses are conveyed along a transmission line to an electrically conductive probe **30**. The electrical pulses are partially reflected when there is a change in the electrical impedance of the fluid **14** to which the probe **30** is exposed. The impedance change is associated with a difference in dielectric strength. "Electrical permittivity" is a technical term indicating the dielectric properties of the fluid **14**. The electrical pulses produced by a time domain reflectometry system are affected by the dielectric constant of the surrounding fluid **14** in which the electrical pulse is traveling. The dielectric constant (permittivity) of the fluid **14** directly affects the propagation velocity of an electromagnetic wave as it travels along the probe **30**. In time domain reflectometry systems, an electromagnetic pulse is propagated into and along the probe **30** which has a known length while measuring the time of arrival and the time of reflection from electrical discontinuities at two known, spaced apart, points. The first known point is where a coaxial cable **75** is attached to the probe **30**. The second known spaced apart point, is a distal end of the probe **30**. Since these locations are both known, it is possible to calculate the propagation velocity of the electromagnetic wave and, as a result, calculate the apparent dielectric constant of the material undergoing tests and to which the probe **30** is exposed. Similarly, changes in the dielectric constant which relate to changes in the fluid **14** adjacent to and surrounding the probe **30** can also be determined. For example, the apparent dielectric constant provides a direct indication of the presence of identifiable types of fluids **14** and condensates **19**.

The pulse emitter **120** which may be incorporated into a TDR is an electronic apparatus that emits electronic pulses (not shown) which are conveyed to the probe **30** through the coaxial cable **75** at a preferred rate of approximately 500 to 800 samples per second depending upon the speed of computation and generating approximately 500 data points per sample. This means the electronic pulses are at increments of approximately 0.76 picoseconds. When the pulse emitter **120** emits a pulse (not shown) the pulse is conveyed along the coaxial cable **75** and to the probe **30** center conductor **60** through the conductor weld pad **71**. The pulse travels along the center conductor **60** whereupon, depending upon the constituents **15**, **16**, **17**, **19** of the surrounding fluid **14** and the respective impedance (dielectric constants) of the constituents **15**, **16**, **17**, **19** to which the probe **30** is exposed, an electrical pulse reflection (not shown) is created when the pulse experiences a change in velocity due to a change in electrical impedance caused by a change in dielectric constant of the fluid **14** within the probe gaps **55** and surrounding the probe **30** active area. The pulse reflection is received from the probe **30** through the coaxial cable **75** and is communicated to the pulse sampler **150** where the reflection is sensed and recorded.

As the dielectric constant properties of the fluid **14** constituents **15**, **16**, **17**, **19** surrounding the probe **30** and within the probe gaps **55** change due to movement of the constituents **15**, **16**, **17**, **19** through the pipe **20**, the velocity and distance traveled by the pulse in the increment of time between any two sequential pulses, changes the apparent length of the probe **30**. The pulse reflection, which indicates the end of the probe **30** or impedance change (the length of the probe in time), is conveyed along the coaxial cable **75** to the pulse sampler **150**. Known computer logic within the computer **170** which is in electronic communication with the pulse emitter **120** and the pulse sampler **150** calculates the "length of the probe in time." Determination of the "length of the probe in time" is empirically representative of the dielectric constant of the fluid constituent **15**, **16**, **17**, **19**.

The computer **170** has a database **172**, which has stored therein, data and information on predetermined known dielectric constants of fluid constituents **15**, **16**, **17**, **19** and predetermined time delays generated by various dielectric constants. The database **172** also has stored therein predetermined known data and information of resonance points of various known volume fraction constituents **15, 16, 17, 17A, 17B, 17C, 17D, 19** and the resonance points of various concentrations of the volume fraction constituents **15, 16, 17, 17A, 17B, 17C, 17D, 19**. The database **172** may also be a correlation or an algorithm wherein information may be correlated and/or compared.

The computer **170** determines the time difference between emission of the electrical pulse into the probe **30** by the pulse emitter **120**, and receipt of the pulse reflection from the probe **30**, by the pulse sampler **150**. The determined time is then correlated by the computer **170**, using the database **172** to known predetermined dielectric constants of known volume fraction constituents **15, 16, 17, 17A, 17B, 17C, 17D, 19** which would similarly generate the determined time difference. The correlation of the determined time difference with information contained within the database **172** permits identification of the volume fraction constituent **15, 16, 17, 17A, 17B, 17C, 17D, 19** by "matching" the determined time difference, with the predetermined known dielectric constant of various known constituents **15, 16, 17, 17A, 17B, 17C, 17D, 19** which allows identification of the constituent **15, 16, 17, 17A, 17B, 17C, 17D, 19**.

The determined time difference between the electrical pulse emission from the pulse emitter **120** into the probe **30**, and receipt of the electrical pulse reflection from the probe **30** by the pulse sampler **150** provides a "length of the probe" measurement which is shared with a detection algorithm within the computer **170** that compares the known "length of the probe" (which correlates to the impedance of the probe **30**) to known dielectric constants, which may vary with salt content, and temperature as detected by the temperature sensor **100** in order to match the determined parameters with a known baseline to identify the volume fraction constituents **15**, **16**, **17**, **19** within the fluid **14**. This first measure is time domain evaluation. It is the behavior of the electrical pulse within the probe **30**, and the resulting length of the probe **30** which allows a first identification of the fluid constituents **15**, **16**, **17**, **19** passing through the grayloc support **80** medial chamber **85**. As the fluid **14** passes around and about the probe **30** and through the gaps **55** between the center conductor **60** and proximate edges of the ground plates **40**, **50**, the pulse reflection, received by the pulse sampler **150** changes as the volume fraction constituents **14**, **15**, **16**, **17**, **19** of the fluid **14** change. The change is caused by the changing electrical impedance and changing dielectric constant of the fluid **14** that is in contact with the probe **30** and immediately surrounding the probe **30**. However, it is known that the dielectric constants of such volume fraction constituents **15**, **16**, **17**, **19** are variable and dependent upon temperature and salt content and therefore using only one measure does not generate consistently reliably accurate results.

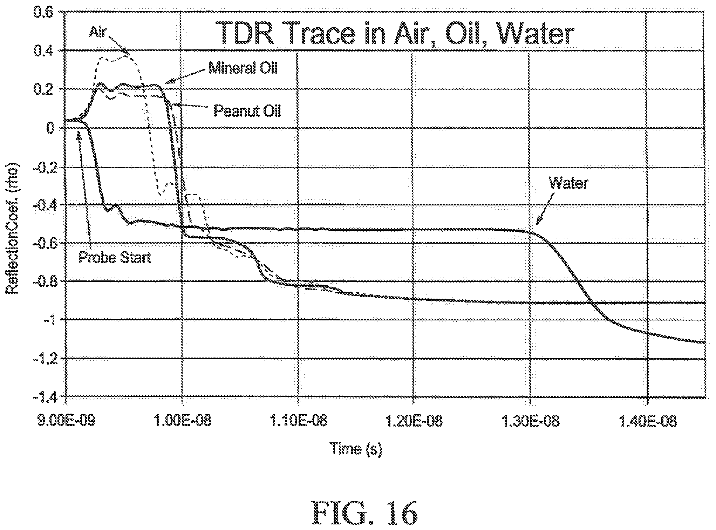

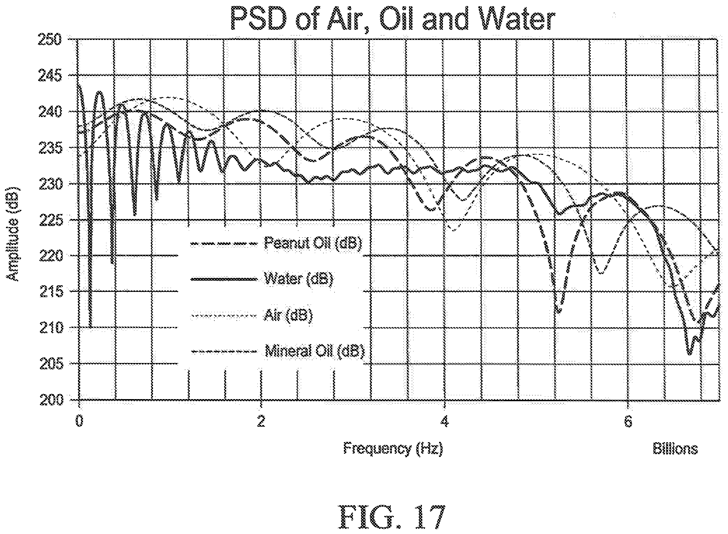

A second, frequency domain analysis takes advantage of the resonance of an electrical signal in the fluid **14** and allows measuring of a volume of the volume fraction constituent **15**, **16**, **17**, **19** within the fluid **14**. By performing a Fourier Transform (FT) of the pulse reflection, a sine wave frequency is determined. The frequency and amplitude of the sine wave signal (Power Spectral Density PSD) as a function of frequency allows different characteristic patterns of the constituents **15**, **16**, **17**, **19** to be identified. By examining the various resonance points as the frequency increases, the distance between the resonance points and the amplitude (strength) of the resonance points provide additional information as to various hydrocarbon constituents within the fluid **14** and allows identification and characterization of those various components, and other components which may be newly appearing in the fluid **14** passing by the probes **30A**, **30B**. Figure 16 shows the combined signals from a probe **30** in water **16**, mineral oil, peanut oil and air. (Peanut oil and mineral oil were used in testing as representative oils to replicate petroleum). Figure 17 shows the FT transform of the same signals taken from the probe **30** in the different fluids **14** showing the Power Spectral Density (PSD) as a function of the frequency. As can be seen, the frequency/amplitude points of water **16**, oil **15**, air and peanut oil are distinctly different from one another, and changes in the relative fractions of the composition (concentrations) of the oil **15** causes a resulting shift in the resonance. The shift in resonance allows a measure of the fraction of each of the volume fraction constituents **15**, **16**, **17**, **19**.

By performing the Fourier Transform (FT) of the reflected electrical pulse received by the pulse sampler **150**, and by performing a Power Spectral Density (PSD) calculation, the frequency and amplitude of the resonance points can be identified.

The FT takes a time-based plot (the determined time delay) and converts the time-based plot into a series of sine waves that duplicate the time history of the electric pulse as a series of frequency based sine waves with the maximums and minimums of the sine waves representing amplitude and resonance points of the volume fraction constituents **15**, **16**, **17**, **19** to which the probe **30** is exposed during the pulse and reflection thereof. The PSD calculation determines the average power, amplitude and frequency of the FT transform. The first resonance point is identifiable because it has a wavelength that is equal to twice the active length of the probe **30**. The relative permittivity of the fluid **14** is calculated by comparing the determined velocity of the pulse in the fluid constituents **15**, **16**, **17**, **19** to the velocity of light in a vacuum using the following relationship between velocity and dielectric:

$$\frac{cf}{c} = \sqrt{1/ef}$$

, where *cf is* the transmission speed of the pulse in the fluid **14**, *c* is the speed of light in a vacuum, and *ef* is the relative permittivity or dielectric constant of the fluid **14**. It is further noted that an inverse of the FT allows recreation of the time history plot.

Figure 16 shows combined time delay signals from a probe **30** exposed to water **16**, oil **15** and air. The time delay shown in Figure 16 is the transit time for the pulse to reach the end of the probe **30** and reflect therefrom. This time delay is proportional to the dielectric constant of the constituents **15**, **16**, **17**, **19** surrounding the probe **30**. Figure 17 shows a graphed Fourier Transform and PSD of the signals shown in Figure 16. Figure 17 also shows the resonant peaks generated by the probe **30** in air, water **16** and oil **15**.

As can be seen in Figure 16, the dielectric constants are all different from one another, and changes in the relative volume fractions **15**, **16**, **17**, **19** causes a shift in the resonance peaks.

As shown in Figures 1 and 2, a second grayloc support **80A** is interconnected with the pipe **20** a known distance **76** downstream from the first grayloc support **80**. The second downstream grayloc support **80A** carries a second probe **30B** that is identical in configuration and function to the first probe **30A**. The second probe **30B** is similarly electronically coupled with a pulse emitter **120** and also with a pulse sampler **150**, or a combined TDR. (Not shown). The pulse emitter **120** and pulse sampler **150** perform the same functions as the previously identified pulse emitter **120** and pulse sampler **150** to determine a time delay between the pulse emission into the probe **30B** and receipt of a pulse reflection from the probe **30B** by the pulse sampler **150**. The determined time delay allows determination of the dielectric constants of the constituents **15**, **16**, **17**, **19** of the fluid **14** by comparison to the known, pre-determined time delay information stored in the database **172** information that is assessable by the computer **170**. Each probe **30A**, **30B** may be, coupled with, a separate pulse emitter **120** and a separate pulse sampler **150** which as noted previously may be combined within a single TDR. (not shown). The computer **170**, and the database **172** accessible thereby, is electronically coupled with both pulse emitters **120** and both pulse samplers **150** (both TDR's) so as to correlate the determined time delays from each probe **30A**, **30B** with the information within the database **172**.

The known distance **76** between the first probe **30A** and the second probe **30B** allows the instant invention to continuously, and in real time, determine the volume of each volume fraction constituent **15**, **16**, **17**, **19** moving through the pipe **20**. Because the computer **170** is electronically coupled with the first probe **30A** and with the first pulse emitter **120**, and the first pulse sampler **150**, and also with the second probe **30B** and the second pulse emitter **120**, and the second pulse sampler **150**, the computer **170** is able to determine a time delay between the first probe's **30A** identification of a specific volume constituent **15**, **16**, **17**, **19** and the second probe's **30B** identification of the same volume constituent **15**, **16**, **17, 19** subsequent to the first probe **30A** identification. Because the interior diameter **23** of the medial channel **28** is known, the total volume of the fluid **14** moving through the pipe **20** by unit of time may be calculated once the velocity of the fluid **14** in the pipe **20** is determined. The time delay between the first probe **30A** identifying a specific volume constituent **15**, **16**, **17, 19** and the second probe **30B** subsequently identifying the same volume constituent **15**, **16**, **17, 19** is used in conjunction with the known distance **76** and known volumetric formulas to determine the volume of identified volume fraction constituents **15**, **16**, **17**, **19** moving through the pipe **20**. The probe's **30A**, **30B** detection of a change in probe length, as described earlier, is indicative of a different volume fraction constituent **15**, **16**, **17**, **19** being identified by the probe **30A**, **30B** and that information, which is communicated to the computer **170** allows identification of the volume constituent **15**, **16**, **17**, **19**, and the volume of the volume of that constituent **15**, **16**, **17**, **19** to be determined.

The time domain evaluation, and the frequency domain evaluation, provide two separate methods to identify volume fraction constituents **15**, **16**, **17**, **19** in the fluid **14** and further allows a determination of a volume of each volume fraction constituent **15**, **16**, **17**, **19** to be determined as the fluid **14** moves through the pipe **20**, on a continuous basis. The frequency domain evaluation further allows the concentration of the various volume fraction constituents **15**, **16**, **17, 19** in the fluid **14** to be determined by correlating the resonance points of the fluid constituents with known resonance points of known constituent concentration within the database **172**.

Each probe **30A**, **30B** has a probe body **31** (Figures 6-10) that is generally rectangular in shape and formed of a metallic alloy and is preferably approximately 1.3 mm (0.050 inches) thick from the first surface **34** to the second surface **35** and approximately 25 mm (1.00 inches) in width from the first edge **37** to the second edge **38**. The probe body **31** is preferably formed entirely of INCONEL® alloy 725 which is highly resistant to the corrosive environment to which the probe body **31** may be exposed during operation. Further, a desirable and durable dielectric oxide coating (not shown) is formed on the probe of body 31 extending entirely thereabout. INCONEL® alloy 718 may also be used, but INCONEL® alloy 725 is preferred. INCONEL® alloy 725 and INCONEL® alloy 718 are available from Megamex Specialty Metals of Humble, Texas.

The method of forming the probe **30**, which carries the durable dielectric oxide coating on its outer surfaces **34**, **35**, includes the steps of cutting the desired probe **30** shape from the desired metallic alloy and then oxidizing cleaning the probe body **31** at approximately 1,750° to 2,000° Fahrenheit in air for one to three hours in order to form the highly electrically resistive oxide surface covering the entire body **31** of the probe **30**. The temperatures used in formation of the oxide coating reduce cracking of the oxide coating and prevents embrittlement caused by grain growth. Following the one to three-hour heat treatment, the probe body **31** is cooled to less than 1,000° Fahrenheit. Subsequently, the probe body **31** is heated in air to 1,325° Fahrenheit for a period of about 8 hours. Thereafter, the probe body **31** is air cooled in an oven to ambient temperature. The heat treatment process forms a chrome alumina oxide coating covering the entire probe body **31** to insulate the probe body **31** in the fluid **14**. The oxide coating is preferably approximately 0.5mm to approximately 3mm thick and is believed to have a chemical composition of approximately CrMoNbTiAl.

It is desirable that the probe body **31**, carrying the chrome alumina oxide coating has an impedance of approximately 90 ohms in air, which allows use of a 90-ohm coaxial cable 75 for interconnection with the pulse emitter **120** and the pulse sampler **150**. The use of a 90-ohm coaxial cable **75** allows the probe **30** to measure 100% water **16**; water **16** containing very little oil **15**; 100% oil **15**; and oil **15** containing very little water **16**. Providing for such a wide range of measurements of water/oil/gas/condensate mixtures allows the probe **30** to measure a full range of "water cuts". Further, the ability to operate at 90 ohms allows the probe **30** to identify drilling fluids (not shown) and components thereof and also identify and measure effective water **16** content within drilling fluids. The probe's **30** the ability to measure water content allows the probe **30** to be used in stationary operations, such as to measure the water **16** content of a standing pool of fluid **14**, such as fuel in a fuel tank (not shown) that may be contaminated with an unknown amount of water **16**. The ability to detect and measure moving/flowing fluids allows the instant invention and probes **30** to be used in the drilling of hydrocarbon producing wells, as well as the use in hydrocarbon producing wells that are in production.

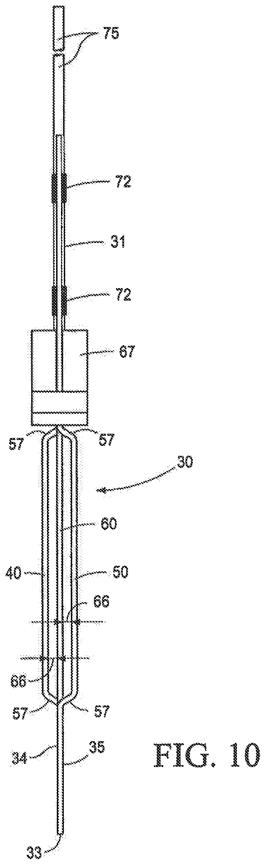

As shown in Figures 9 and 10, a second design of probe **30** is also contemplated herein. This second probe **30** design is intended to reduce potential (clogging) due to particulates and solids within the fluid **14** moving through the medial channel **28** of the pipe **20** and the grayloc supports **80** and is particularly useful for use in producing wells where the slugs **250** of liquid may contain solids and particulates (not shown), such as, but not limited to sand. In the second contemplated design (Figures 9, 10) the first ground plate **40** is offset toward the first surface **34** in the thickness dimension 36 relative to the center conductor **60** defining a gap **55** of approximately 2 mm (0.080 inches) between an inner proximate edge **44** of the first ground plate **40** and the center conductor **60**. Similarly, the second ground plate **50** is offset in the thickness dimension **36** toward the second surface **35** by a distance of approximately 2 mm (0.080 inches) to define a gap **55** between the proximate inner edge **54** of the second ground plate **50** and the center conductor **60**. The mutual perpendicular and opposite offsetting of the first ground plate **40** and the second ground plate **50** relative to the center conductor **60** is facilitated by bends **57** at a bottom portion of the offset portion (the active length **66**), and at an upper portion of the offset portion so that only the active portion **66** of the probe body **31** is laterally offset to allow fluid **14** to flow through the gap **55**. (Figure 10). In other aspects, the second probe design (Figure 10) is the same as that of the first probe design (Figure 6).

The first ground plate **40** is offset relative to the elongated center conductor **60** and the generally planar body **31** in a direction opposite the second surface **35** and toward the first surface **34** so as to be spaced apart from the proximate laterally outer edge portion of the elongated center conductor **60** in two mutually perpendicular directions, (in the thickness dimension **36**) and the second ground plate **50** is offset relative to the elongated center conductor **60** and the generally planar body **31** in a direction opposite the first surface **34** and toward the second surface **35** so as to be spaced apart from the proximate laterally outer edge portion of the elongated center conductor **60** in two mutually perpendicular directions (in the thickness dimension).

The first ground plate **40** is offset in a thickness dimension toward the first surface **34** and opposite the second surface **35** relative to the center conductor **60** a distance of approximately 2 mm (0.080 inches), and the second ground plate **50** is offset in a thickness dimension toward the second surface **35**, opposite the first surface **34**, relative to the center conductor **60** a distance of approximately 2 mm (0.080 inches). Further, the first surfaces 34 of the first ground plate **40**, the elongated center conductor **60** and the second ground plate **60** are parallel to one another but are not coplanar with one another, and the second surfaces **35** of the first ground plate **40**, the elongated center conductor **60** and the second ground plate **50** are parallel to one another, but are not coplanar with one another. Further still, the first ground plate **40** and second ground plate **50** are offset from the center conductor **60** a distance of between approximately 1.3 mm (0.051 inches) and 2.8 mm (0.110 inches) and a width of the elongated gap **55** between an inner edge of the first ground plate **40** and an inner edge of the second ground **50** plate is about 7.9 mm (0.310 inches).

In a third possible configuration (Figures 24, 25) the EFP probe **30** is a "through probe" design such that there is no reflection signal generated by a terminal end of the probe **30**. Rather, a length of coaxial cable **75** has a first end that is electrically interconnected with the second end **33** of the probe **30** and a second end of the elongated center conductor **60**. The coaxial cable **75** has a second opposing end (not shown) that is electrically interconnected with the pulse sampler **150** which effectively creates an "endless loop" which prevents the creation of any terminal-end "reflection signal". The "through probe" configuration (Figures 24, 25) has the added benefit of enhancing contrast and lengthening the signal to provide enhanced ability to evaluate the resonance points and permittivity curve of the constituents **15**, **16**, **17**, **17A**, **17B**, **17C**, **17D**, **19** surrounding the probe **30**.

The third configuration of the probe **30** (Figures 24, 25) requires use of a two port TDR (not shown) having one port (not shown) that sends the electrical pulse signal (not shown) (functioning as the pulse emitter **120**) and a second (separate) port (not shown) (functioning as the pulse sampler **150**) that receives the electrical pulse signal so that any signal confusion is removed, or at least minimized. The dual port system allows determination of phase relationships and the complex permittivity (real and imaginary) curves more efficiently than using the reflected single port system including more subtle variations in the materials being examined.

A complex permittivity curve describes the electrical permittivity of a material and is a property of the material independent of the EFP system and is related to the concept of dielectric constant and the complex permittivity curve for a given material may change with density and temperature.

All materials have electrical permittivity. Oil **15** and water **16** and natural gas **17** and condensates **19** have distinct complex permittivity curves at any density and temperature encountered in extraction operations. A measured complex permittivity curve for an oil-water-gas mixture can be used to determine the oil-water-gas ratio.

Complex permittivity for a mixture can be calculated from scattering parameters of the probe **30** that is submerged in the mixture. The scattering parameters of the probe are a function of the probe **30** geometry and the complex permittivity of the fluid **14** that surrounds it. By knowing the probe **30** geometry and the scattering parameters is sufficient to make an estimate of the complex permittivity of the fluid **14** and its constituents.

The instant method contains a database **172** of known complex permittivity curves for oil **15** and water **16** and natural gas **17** and condensates **19** at ranges of temperatures and pressures.

Scattering parameters are a representation of a passive electrical component, such as an EFP probe **30**. A complete set of scattering parameters totally describes the electrical behavior of a component, (the probe **30**) and there are different equivalent representations of scattering parameters. One preferred representation is as a time-domain response trace for a unit electric impulse. Another preferred representation is a frequency domain specification of gain and phase shift for inputs at given frequencies.

When represented in the frequency domain, the scattering parameter is a complex number, with the amplitude giving the gain from input to output, and the phase angle of the value matching the phase shift of the output relative to the input.

When the scattering parameters are known, and the input signals are known, the output signals can be calculated by convolving the input signals with the scattering parameters.

With the EFP probe **30**, the input pulse signal is known by calibration of the pulse generator **150**, and the output is measured. The scattering parameter can then be calculated by de-convolving the input signal from the received signal.

The design of the EFP probe **30** ensures that the scattering parameters of the probe **30** are predictably related to the complex permittivity of the fluid **14** to which the probe **30** is exposed.

Scattering parameters are not properties of the fluid **14**, but electrical properties of the EFP probe **30** that can be used to calculate a complex permittivity curve of the fluid **14**. Oil **15** and water **16** and natural gas **17** do not have scattering parameters. However, the scattering parameters of the EFP probe **30** are significantly different when submerged in oil **15** or water **16** or natural gas **17**.

Like scattering parameters, resonance points are an electrical property of the EFP probe **30**. They are not a property of the fluid **14**. Oil **15** and water **16** and natural gas **17** and condensates **19** do not have resonance points.

The EFP probe **30** is designed so that its resonance points are significantly different when in **oil 15** versus when in water 16 versus when in natural gas 17.

Resonance points are frequencies which resonate with the EFP probe **30**. They are near integer or half-integer (1.5, 2.5) multiples of some lowest frequency. The small deviations from one resonance point to the next can be used to estimate the amplitude of the complex permittivity at the frequency of the resonance points.

Resonance points can be determined and measured from an EFP pulse reflected or transmitted signal by analysis of the signal transformed to the frequency domain using an FFT.

Three different techniques are used to calculate dielectric constant and complex permittivity. These techniques can be used with each of the contemplated probe **30** designs.

Each technique is more powerful, but more complicated than the one before. Each can be used simultaneously on the same EFP signal.

The terms Dielectric Constant and Permittivity are related, and to some extent, are the same thing. The dielectric constant is also called the Relative Permittivity. For a material, the relative permittivity is the ratio between the absolute permittivity of the material and the permittivity of free space.

Relative permittivity can be measured by measuring the capacitance between two plates with the material there-between. The higher the dielectric, the higher the capacitance.

For an AC signal, the permittivity of a material typically varies between frequencies. The dielectric constant, which is only the relative permittivity at 0 Hz and does not say much about the electrical properties of the material at higher frequencies.

Also, with an AC signal, energy can be lost to the material. The combination of a lossless component and a lossy component of electrical permittivity is represented as a complex number, with the lossy component as the imaginary term.

The speed of the pulse through the probe **30** is inversely related to the dielectric constant of the fluid **14** surrounding the probe **30**. As the dielectric constant changes, the time for an electric pulse to leave the EFP and return will also change.

If a sine wave of a single frequency were sent continuously from the EFP to a probe, the wave would reflect from two places: the start of the probe and the end of the probe 30. The reflections would be sine waves of the same frequency.

The reflected sine waves add together and appear as a single sine wave at the EFP. At certain frequencies, the reflecting waves will cancel each other out. This phenomenon is known as destructive interference. At these frequencies, the apparent amplitude of the reflected pulse drops suddenly.

The wavelength of an electric pulse at a given frequency is a function of the speed of the pulse. The speed of the pulse is inversely related to the permittivity at that frequency. From a divisor of a resonance point it is possible calculate the wavelength. From the wavelength and the frequency of the resonance point it is possible to calculate speed, and therefore calculate permittivity.

Because the resonance point technique is a single frequency measurement, the result is frequency dependent permittivity. Because there are multiple resonance points the permittivity is determined at a number of different frequencies.

**OPERATION**

Having described the method for identifying and characterizing a condensate entrained in a fluid, the operation may be understood.

A source of fluid **13** is provided and is interconnected with a pipe **20** defining the medial channel **28** to provide fluid **14** moving therethrough, the fluid **14** having a volume fraction constituent **15**, **16**, **17**, **17A**, **17B**, **17C, 17D** and condensate **19** that is desired to be identified and characterized and measured, and wherein the volume fraction constituent **15**, **16**, **17**, **17A**, **17B**, **17C**, **17D**, **19** has previously calculated and known dielectric constant, and a previously calculated and known resonance points, and wherein information about the previously calculated and known dielectric constant and previously calculated and known resonance points of the volume fraction constituent **15**, **16**, **17**, **19** is stored in, and is accessible from a database **172**.

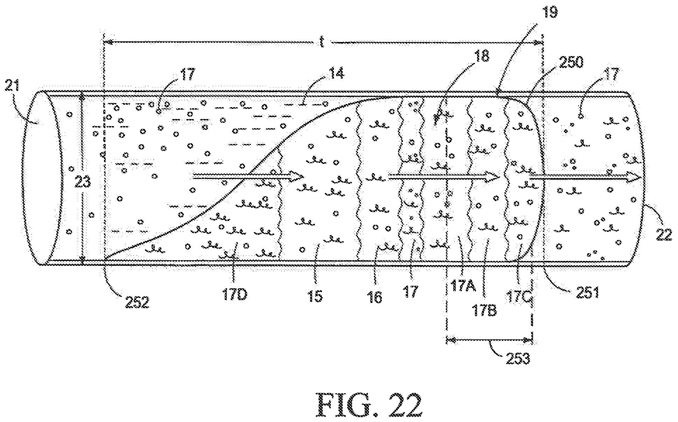

The fluid **14** enters the pipe **20** from the source of fluid **13** and passes through the pipe **20** as a liquid slug **250** (See Figure 22) that may be both transient and periodic. The liquid slug **250** has a leading edge portion **251** and a trailing edge portion **252** and is comprised of a variety of condensate **19** and natural gas **17** such as, but expressly not limited to, ethane **17A**, butane **17B**, pentane **17C** and propane **17D**. The condensate **19** and natural gas **17** may further be in the form of an emulsion **18** that is mixed with water **16** and/or oil **15** so that the condensate **19** is entrained within the fluid **14**.

The slug **250** is transient in the pipe **20** and commonly has a duration of about two hundred (200) seconds between the leading edge portion **251** and the trailing edge portion **252** when moving through the conduit/pipe **20**. The concentration of condensate **19** and volume fraction constituents **17**, **17A**, **17B**, **17C**, **17D** is typically highest/greatest at or near the leading edge portion **251** of the liquid slug **250** and therefore the measurements/sampling of the constituents of the slug **250** is preferably made at or near the leading edge portion **251** of the slug **250** as the slug passes through the pipe **20** and passed the EFP probes **30A**, **30B**. The measurements performed by the probes **30A**, **30B** are performed continuously, including continuously throughout the passage of the liquid slug **250** past the probes **30A**, **30B**. The most accurate measurements are taken in a period **253** (Figure 22) that has a duration of approximately two (2) to seven (7) seconds following the leading edge portion **251** of the slug **250**. It is proximate the leading edge portion **251** of the slug **250** that the concentrations of the condensates **19** and constituents **17A-D** are the highest with the least dissipation by methane gas bubbles. (not shown).

First probe **30A** is exposed at least in part to the fluid **14** moving through the pipe **20**, the first probe **30A** having a known active length, and the first probe **30A** is positionally maintained within a medial chamber **85** defined by a grayloc support **80** communicating with the medial channel **28** of the pipe **20**, so that the slug **250** and fluid **14** and condensate **19** flows therethrough and thereabout and there-past the first probe **30A**.

Second probe **30B** is also exposed at least in part to the fluid **14** moving through the pipe **20**, a known distance **76** downstream of the first probe **30A**, the second probe **30B** having an known active length, and the second probe **30B** is positionally maintained within a medial chamber **85** defined by a second grayloc support **80A** that also communicates with the medial channel **28** of the pipe **20**, a known distance **76** downstream of the first grayloc support **80** so that the slug **250**, the fluid **14** and the condensate **19** flows therethrough, and thereabout and there-past the second probe **30B**.

A back pressure regulator **110** communicating with the medial channel **28** of the pipe **20** may maintain fluid pressure about the probes **30A**, **30B** at a pressure at least equal to the pressure of the source of the fluid **13** to prevent boiling of the fluid **14** within the pipe **20** to prevent formation of steam within the pipe **20**, because steam has a dielectric constant that is similar to the dielectric constant of natural gas **17** and condensate **19** which would make it difficult to distinguish between a volume of natural gas **17** and condensate **19** and a volume of steam.

A first electrical pulse emitter **120** electronically generates an electrical pulse which is conveyed to the first probe **30A** through the coaxial cable **75**. The electrical pulse then generates an electrical pulse reflection upon interacting with a changed electrical impedance (which is indicated as an end of the first probe **30A**) and which is caused by a change in sensed dielectric constant of the volume fraction constituent **15**, **16**, **17**, **19** to which the first probe **30A** is exposed. The first electrical pulse sampler **150** receives and senses of the electrical pulse reflection.

Similarly, the second electrical pulse emitter **120** electronically generates an electrical pulse which is conveyed to the second probe **30B** through the coaxial cable **75**. The electrical pulse similarly generates an electrical pulse reflection upon interacting with the changed electrical impedance (which is indicated as an end of the second probe **30B**) and which is caused by a change in sensed dielectric constant of the volume fraction constituent **15**, **16**, **17**, **19** to which the second probe **30B** is exposed. The second electrical pulse sampler **150** receives and senses of the electrical pulse reflection.

If the third contemplated configuration of probe **30** (Figures 24, 25) is utilized for the first probe **30A** and the second probe **30B**, the change in electrical impedance of the fluid **14** is detected as a reflection signal that is electrically communicated to the interconnected pulse sampler **150**. A portion of the original electrical pulse signal (not shown) communicates through the entire active length of the probe **30** and thereafter to the interconnected pulse sampler **150** via the coaxial cable **75**. The difference is distance traveled (measured as a time delay) by the electrical pulse reflection signal (due to the change in impedance of the fluid **14**/condensate **19**) and the electrical pulse pass through signal enhances the signal contrast.

The computer **170** is electronically coupled with the first probe **30A**, the first electrical pulse emitter **120**, the first electrical pulse sampler **150** and the database **172**. The computer **170** determines a time delay between the electrical pulse emission into the first probe **30A** and receipt of the sensed electrical pulse reflection from the first probe **30A**.

The computer **170** is also electronically coupled with the second probe **30B**, the second electrical pulse emitter **120**, the second electrical pulse sampler **150** and the database **172**. The computer **170** also determines a time delay between the electrical pulse emission into the second probe **30B** and receipt of the sensed electrical pulse reflection from the second probe **30B**.

The computer **170** performs the time domain evaluation by correlating and comparing the determined time delay between pulse emission and pulse reflection receipt to the information within the database **172** to match the determined time delay to similar time delays generated by known dielectric constants, and then the computer **170** correlates the identified dielectric constant to known and previously determined volume fraction constituents **15**, **16**, **17**, **19** having such dielectric constants. The computer also performs the frequency domain evaluation by determining/calculating the resonance points of the volume fraction constituents **15**, **16**, **17** and condensate **19** and concentrations thereof in the fluid **14** by applying a Fast Fourier Transform (FFT) to the previously determined time delay. A Power Spectral Density (PSD) evaluation is then made of the calculated resonance points by the computer **170** to determine the average power, amplitude and frequency of the volume fraction constituents **15**, **16**, **17** and condensate **19**. The computer **170** then correlates the resonance points resulting from the FFT and PSD to the previously calculated and known resonance points as provided in the database **172** as a second measure to identify the volume fraction constituents **15**, **16**, **17** and condensates **19** entrained in the fluid **14** and to measure the volume of the volume fraction constituents **15**, **16**, **17** and condensate **19** in the fluid **14**. The resonance points may also be correlated with the known permittivity curves stored in the database **172** so as to identify the volume fraction constituents **15**, **16**, **17** and condensates **19** entrained in the fluid **14** and to measure the volume of the volume fraction constituents **15**, **16**, **17** and condensate **19** in the fluid **14**.

A first output (not shown) is generated by the first probe **30A** when a volume fraction constituent **15**, **16**, **17** and condensate **19** is sensed by the first probe **30A**, and a second output (not shown) is generated by the second probe **30B** when the same volume fraction constituent **15**, **16**, **17** and condensate **19** is subsequently sensed by the second probe **30B**. The first and second probe outputs (not shown) are communicated to the computer **170** through the coaxial cable **75** wherein the computer **170** uses the time delay between the first probe **30A** output and the second probe **30B** output to determine the velocity of the volume fraction constituents 15, **16**, **17** and condensate **19** moving through the conduit or pipe **20**. The externally mounted auxiliary fluid flow measurement device **90A** communicating with the pipe **20** downstream of the second probe **30B** and electrically communicating with the computer **170** may provide additional, and independent, volumetric flow data to more accurately determine and measure volumes of the fluid **14** and constituents and condensate **19**.

Display **171** is electronically coupled with the computer **170** and receives the identification of the volume fraction constituents **15**, **16**, **17**, **17A**, **17B**, **17C**, **17D** and condensate 19 and the volume fraction **15**, **16**, **17** volume calculation data from the computer **170** to generate a user perceivable output (not shown) which identifies the volume fraction constituents **15**, **16**, **17** and condensate **19** entrained in the fluid **14** and the volume thereof moving through the pipe **20** continuously and in real time.

The instant invention also provides a method for identifying and characterizing the volume fraction constituents **15**, **16**, **17** of a fluid **14** and condensates **19** entrained therein. The method is first initiated by providing a source of fluid **13** which communicates with the pipe **20** that defines a medial channel **28** for the fluid **14** to move therethrough. The fluid **14** has a volume fraction constituent **15**, **16**, **17** and condensate **19** and each volume fraction constituent **15**, **16**, **17** and condensate **19** has a previously calculated and known dielectric constant and previously calculated and known resonance points.

The database **172**, which is assessable by the computer **170**, has stored assessable information about the previously calculated and known dielectric constant of each volume fraction constituent **15**, **16**, **17** and condensate **19** and stored assessable information about the previously calculated and known resonance points of each volume fraction constituent **15**, **16**, **17** and condensate **19**, and each volume fraction constituent at various concentrations.

The first probe **30A** is positionally maintained within the upstream grayloc support 80, and the first probe **30A** is exposed, at least in part, to the fluid **14** moving through the medial channel **28** of the pipe **20** and through the upstream grayloc support **80**. The second probe **30B** is similarly positionally maintained within a second grayloc support **80A**, and the second probe **30B** is exposed, at least in part, to the fluid **14** moving through the medial channel **28** of the pipe **20** and through the second grayloc support **80A** downstream a known distance **76** from the first probe **30A**.

The back pressure regulator **110** which communicates with the medial channel **28** of the pipe **20** maintains fluid pressure within the medical channel **28** and about the first and second probes **30A**, **30B** respectively, at a pressure at least equal to the pressure of the source of fluid **13** to prevent boiling of the fluid **14** within the medial channel **28** of the pipe **20**.

The first electrical pulse emitter **120** electronically generates an electrical pulse that is conveyed to the first probe **30A** through the coaxial cable **75**. The electrical pulse is conveyed into the first probe **30A** and generates an electrical pulse reflection when the electrical pulse travels the entire active length of the first probe **30A**, or earlier interacts with a changed electrical impedance or a changed dielectric constant of a volume fraction constituent **15**, **16**, **17** and/or condensate **19** to which the first probe **30A** is at least partially exposed. The pulse reflection is received by the first electrical pulse sampler **150** that is electronically coupled with the first probe **30A** by the coaxial cable **75**.

Similarly, the second electrical pulse emitter **120** electronically generates an electrical pulse that is conveyed to the second probe **30B** through the coaxial cable **75**. The electrical pulse is conveyed into the second probe **30B** and a generates an electrical pulse reflection when the electrical pulse travels the entire active length of the second probe **30B** or earlier interacts with a changed electrical impedance or a changed dielectric constant of a volume fraction constituent **15**, **16**, **17** and/or condensate **19** to which the second probe **30B** is at least partially exposed. The pulse reflection is received by a second electrical pulse sampler **150** that is electronically coupled with the second probe **30B** by the coaxial cable **75**.

The computer **170** is electronically coupled with the probes **30A**, **30B** the electrical pulse emitters **120**, the electrical pulse samplers **150** and the database **172**.

The computer **170** determines a time delay between the electrical pulse emission into each probe **30A**, **30B** and receipt of the electrical pulse reflections from each probe **30A**, **30B**.

The computer **170** correlates the determined time delay between the electrical pulse emission into each probe **30A**, **30B**, and receipt of the electrical pulse reflection from the respective probe **30A**, **30B** to the information stored within the database **172** of known time delays generated by known dielectric constants of known volume fraction constituents **15**, **16**, **17** and condensate **19** to provide a measure to identify the volume fraction constituents **15**, **16**, **17** and condensate **19** entrained within the fluid **14**.

The computer **170** applies a Fast Fourier Transform (FFT) to the determined time delay to generate a sine wave frequency based upon the determined time delay. The computer **170** also calculates the Power Spectral Density (PSD) of the generated sine wave frequency to determine the average power, amplitude and frequency of the sine wave to identify resonance points. The computer **170** correlates the frequency from the Fast Fourier Transform (FFT) and the resonance points of the PSD to the database **172** of known resonance points of known volume fraction constituents **15**, **16**, **17** and condensate **19** to provide another measure to identify the volume fraction constituents **15**, **16**, **17** and condensate **19** within the fluid **14** and also to measure the volume of the volume fraction constituents **15**, **16**, **17** and condensate **19** entrained in the fluid **14**.

A first output (not shown) is generated by the first probe **30A** when a volume fraction constituent **15**, **16**, **17** and condensate **19** is sensed by the first probe **30A** and identified by the computer **170**, and a second output (not shown) is generated by the second probe **30B** when the same volume fraction constituent **15**, **16**, **17** and condensate **19** is subsequently sensed by the second probe **30B** and identified by the computer **170**.

The volume of each volume fraction constituent **15**, **16**, **17** and condensate **19** moving through the pipe **20** is calculated by using the determined time delay between the first probe **30A** output and the second probe **30B** output by calculating the velocity of the sensed volume fraction constituent **15**, **16**, **17** and condensate **19** moving the known distance **76** and using the known interior diameter **23** of the pipe **20**.

Display **171** which is electronically coupled with the computer **170** and which receives the identification of the volume fraction constituent **15**, **16**, **17** and condensate **19**, and the first probe **30A** output (not shown) and the second probe **30B** output (not shown) and the correlation of resonance points of the volume fraction constituents **15**, **16**, **17** and condensate **19** generates a user perceivable output (not shown) which identifies each volume fraction constituent **15**, **16**, **17** and condensate **19** entrained in the fluid **14**, and the volume thereof moving through the pipe **20** on a real-time and continuous basis.

The instant inventive method is also usable with a probe **30** that is a through probe.

**Claims**

1.  A method for identifying, and characterizing a periodic release of a given condensate (19) which is entrained within a source of a fluid (13), comprising:

    providing a source of a fluid (13) having a given composition which includes a major volume fraction constituent, and wherein at least one condensate (19) is periodically released, and is then entrained within the source of the fluid (13), and wherein the major volume fraction constituent, and the at least one condensate (19) each have a previously determined, and known, dielectric constant, and a previously determined, and known, resonance point;
    providing a database (172) having accessible, and stored information about the previously determined, and known dielectric constants of the major volume fraction constituent, and/or the at least one condensate (19), and accessible and stored information about the previously determined, and known resonance points of given concentrations of the major volume fraction, constituent and/or the at least one condensate (19);
    providing a probe (30,30A,30B) which is exposed, at least in part, to the source of fluid (13), and which further has a known length dimension;
    providing an electrical pulse emitter (120) which, when energized, generates a given electrical pulse which is electrically delivered to the probe (30,30A,30B), and wherein the electrical pulse electrically travels along the known length dimension of the probe (30,30A,30B), and further generates an electrical pulse reflection;
    providing an electrical pulse sampling device (150) which is electrically coupled in electrical pulse receiving, and sensing relation relative to the probe (30,30A,30B);
    providing a computer (170) which is electrically coupled with the probe (30,30A,30B), the electrical pulse emitter (120), the electrical pulse sampling device (150), and the database (172), and wherein the computer (170) determines a time period which elapses between the electrical pulse emission sent into the probe (30,30A,30B), and the receipt of the sensed electrical pulse reflection received from the probe (30,30A,30B), and wherein the resonance point of the major volume fraction constituent, and the resonance point of the at least one condensate (19) are individually calculated by the computer (170) from the determined time periods, and/or the computer (170) correlates the determined time period to the previously determined, and known, dielectric constants, and wherein the computer (170) then correlates the calculated resonance points of the major volume fraction constituent, and the at least one condensate (19), as provided in the database (172), so as to identify a characteristic of the major volume fraction constituent, and the at least one condensate (19) which is entrained within the source of fluid (13);
    providing a user interface which is electronically coupled with the computer, and which further generates a user perceivable output which identifies the at least one characteristic of the major volume fraction constituent, and the at least one condensate, respectively;
    wherein the condensate (19) which is entrained within the source of the fluid (13) is transiently, and periodically released, and wherein the method further comprises the step of measuring the transient and periodic release of the fluid and condensate (19) over a given time period;
    wherein the transient, and periodic release of the condensate (19), and which further is entrained with the source of the fluid (13), takes place over a time period of less than about 200 seconds;
    the method being **characterized in that** it further comprises electrically sampling the source of the fluid (13) having the given composition, and which includes the major volume fraction constituent, and the at least one condensate (19), during a time period when the major volume fraction constituent, which includes the at least one condensate (19), has the least concentration of a source of methane gas; and wherein the periodic and transient release of the fluid entraining the condensate (19) has a leading edge, and further contains water, and at least one hydrocarbon, each having a predetermined and known resonance point and a predetermined and known dielectric constant, and wherein the method further comprises measuring the volume fraction of the water, and the volume fraction of the at least one hydrocarbon in the periodic and transient release of the fluid entraining the condensate (19) near the leading edge thereof, by employing electric field perturbation which is based upon the time domain reflectometry.

2. A method as claimed in claim 1, and further comprising:

    providing an elongated conduit (20) having an internal bore (28) which has a predetermined, substantially uniform, inside diametral dimension;

    providing two electric field perturbation probes (30A,30B), and positioning each of the electric field perturbation probes (30A,30B), at least in part, within the bore (28) of the elongated conduit (20), and at a known, and predetermined distance, one relative to the other;

    providing a fluid pressure sensor which is mounted in a fluid pressure sensing relationship relative to the internal bore (28) of the elongated conduit (20), and which further generates a fluid pressure signal;

    providing a temperature sensor (100) which is mounted in temperature sensing relation relative to the internal bore (28) of the elongated conduit (20), and which further generates fluid temperature signal;

    electrically coupling the two electric field perturbation probes (30A,30B), fluid pressure sensor, and temperature sensor (100) in a signal transmitting relationship relative to the computer (170);

    delivering the source of the fluid (13) which includes the major volume fraction constituent, and the at least one condensate (19), into the internal bore (28) of the elongated conduit (20);

    electrically sampling, with the computer (170), each of the respective two electric field perturbation probes (30A,30B), fluid pressure, and/or temperature sensor signals; and

    correlating the signals received from the at least two electric field perturbation probes (30A,30B), the pressure sensor and/or temperature sensor (100), with the computer (170), so as to provide a characterization of the source of the fluid (13).

3. A method as claimed in claim 2, and wherein each of the electric perturbation sensors are located at predetermined, spaced apart, sensing stations which are located along the elongated conduit (20); and

    electrically sampling with the computer (170), at each of the sensing stations, at a speed of about 500 frames per second.

4. A method as claimed in 3, and further comprising: calculating, with the computer (170), a flow velocity of the source of the fluid (13) through the internal bore (28) of the elongated conduit (20) from the signals received from the two electric field perturbation probes (30A,30B), and the temperature and fluid pressure sensors.

5. A method as claimed in 4, and further comprising:

    characterizing, with the computer (170), the average composition of the source of the fluid (13) in the region of the respective, spaced, sensing stations by utilizing the signals received from the respective, electric field perturbation sensors; and

    estimating, by utilizing the computer (170), a total flow volume of the source of the fluid (13), and a fractional volume of the at least one hydrocarbon which is entrained with the source of the fluid (13).

6. A method as claimed in claim 5, and further comprising:

    calculating, with the computer (170), an approximate fluid density of the source of the fluid (13), by utilizing the signals received from the temperature and pressure sensors, during a given sampling time; and

    providing an auxiliary, and externally mounted fluid flow measurement device (90A) and coupling the auxiliary, and externally mounted fluid flow measurement device (90A) in a signal transmitting relationship relative to the computer (170);

    delivering the source of fluid (13) to the auxiliary, and externally mounted, fluid flow measurement device (90A), and generating a signal with the auxiliary and externally mounted fluid flow measurement device (90A) which is transmitted to the computer (170);

    measuring the fluid flowing movement of the source of the fluid (13) through the auxiliary, and externally mounted, fluid flow measurement device (90A);

    estimating the total flow of the source of fluid (13), with the computer (170), by utilizing the signal generated by the auxiliary, and externally mounted, fluid flow measurement device (90A); and

improving the estimated calculation of the total flow volume of the source of the fluid (13), and the fractional volume of the at least one hydrocarbon which is entrained with the source of the fluid (13), by utilizing the estimated total flow of the source of fluid (13), and which is detected by the auxiliary, and externally mounted fluid flow measuring device (90A), by employing the computer (170).

7. A method as claimed in claim 1, and further comprising:

applying a Fourier Transform calculation to the sensed electrical pulse reflection received from the probe (30,30A,30B), and which is used to determine a resonant frequency and resonance point of at least one of the volume fraction constituents.

8. A method as claimed in claim 7, and further comprising:

applying a Power Spectral Density (PSD) calculation, by means of the computer (170), to the Fourier Transform (FT) frequency so as to determine an amplitude, and strength of at least one of the given resonance points.

9. A method as claimed 8, and wherein the volume fraction constituent is a multiplicity of volume fraction constituents.

10. A method as claimed in claim 9, and wherein the multiplicity of volume fraction constituents includes a liquid and a gas.

## Patentansprüche

1. Verfahren zum Identifizieren und Charakterisieren einer periodischen Freisetzung eines gegebenen Kondensats (19), das in einem Fluid (13) mitgeführt wird, welches Folgendes aufweist:

Bereitstellen einer Fluidquelle bzw. eines Fluids (13) mit einer gegebenen Zusammensetzung, das einen Hauptvolumen-Bestandteil enthält, wobei mindestens ein Kondensat (19) periodisch freigesetzt und dann in der Fluid (13) mitgeführt wird, und wobei der Hauptvolumen-Bestandteil und das mindestens eine Kondensat (19) jeweils eine zuvor bestimmte und bekannte Dielektrizitätskonstante und einen zuvor bestimmten und bekannten Resonanzpunkt aufweisen;

Bereitstellen einer Datenbank (172) mit zugänglichen und gespeicherten Informationen über die zuvor bestimmten und bekannten Dielektrizitätskonstanten des Hauptvolumen-Bestandteils und/oder des mindestens einen Kondensats (19) und zugänglichen und gespeicherten Informationen über die zuvor bestimmten und bekannten Resonanzpunkte von gegebenen Konzentrationen des Hauptvolumen-Bestandteils und/oder des mindestens einen Kondensats (19);

Bereitstellen einer Sonde (30, 30A, 30B), die zumindest teilweise dem Fluid (13) ausgesetzt ist und die des Weiteren eine bekannte Längenabmessung aufweist;

Bereitstellen eines Senders (120) für elektrische Impulse, der, wenn er erregt wird, einen gegebenen elektrischen Impuls erzeugt, der der Sonde (30, 30A, 30B) elektrisch zugeführt wird, wobei sich der elektrische Impuls elektrisch entlang der bekannten Längenabmessung der Sonde (30, 30A, 30B) bewegt und des Weiteren eine elektrische Impulsreflexion erzeugt;

Bereitstellen einer Vorrichtung (150) zum Abtasten elektrischer Impulse, die in Bezug auf die Sonde (30, 30A, 30B) elektrisch gekoppelt ist, um elektrische Impulse zu empfangen und zu erfassen;

Bereitstellen eines Computers (170), der elektrisch mit der Sonde (30, 30A, 30B), dem Senders (120) für elektrische Impulse, der Vorrichtung (150) zum Abtasten elektrischer Impulse und der Datenbank (172) gekoppelt ist, wobei der Computer (170) eine Zeitspanne bestimmt, die zwischen der in die Sonde (30, 30A, 30B) gesendeten Elektroimpulsemission und dem Empfang der abgetasteten Elektroimpulsreflexion, die von der Sonde (30, 30A, 30B) empfangen wird, verstreicht, und wobei der Resonanzpunkt des Hauptvolumen-Bestandteils, und der Resonanzpunkt des mindestens einen Kondensats (19) von dem Computer (170) aus den ermittelten Zeitspannen individuell berechnet werden und/oder der Computer (170) die ermittelte Zeitspanne mit den zuvor ermittelten und bekannten dielektrischen Konstanten korreliert, und wobei der Computer (170) dann die berechneten Resonanzpunkte des Hauptvolumen-Bestandteils und des mindestens einen Kondensats (19), wie sie in der Datenbank (172) bereitgestellt werden, korreliert, um eine Eigenschaft des Hauptvolumen-Bestandteils und des mindestens einen Kondensats (19) zu identifizieren, das in dem Fluid (13) mitgeführt wird;

Bereitstellen einer Benutzerschnittstelle, die elektronisch mit dem Computer gekoppelt ist und die des Weiteren eine von dem Benutzer wahrnehmbare Ausgabe erzeugt, die das mindestens eine Merkmal des Hauptvolumen-Bestandteils bzw. des mindestens einen Kondensats identifiziert;

wobei das Kondensat (19), das in dem Fluid (13) mitgeführt wird, vorübergehend und periodisch freigesetzt wird, und wobei das Verfahren des Weiteren den Schritt des Messens der vorübergehenden und periodischen Freisetzung des Fluids und des Kondensats (19) über eine gegebene Zeitspanne umfasst;

wobei die vorübergehende und periodische Freisetzung des Kondensats (19), das des Weiteren mit dem Fluid (13) mitgeführt wird, über einen Zeitraum von weniger als etwa 200 Sekunden erfolgt;

wobei das Verfahren **dadurch gekennzeichnet ist, dass** es des Weiteren das elektrische Abtasten des Fluids (13) mit der gegebenen Zusammensetzung, die den Hauptvolumen-Bestandteil enthält, und des mindestens einen Kondensats (19) während eines Zeitraums umfasst, in dem der Hauptvolumen-Bestandteil, der das mindestens eine Kondensat (19) enthält, die geringste Konzentration von Methangas aufweist; und

wobei die periodische und vorübergehende Freisetzung des Fluids, das das Kondensat (19) mit sich führt, einen vorderen Rand aufweist und des Weiteren Wasser und mindestens einen Kohlenwasserstoff enthält, die jeweils einen vorbestimmten und bekannten Resonanzpunkt und eine vorbestimmte und bekannte Dielektrizitätskonstante aufweisen, und wobei das Verfahren des Weiteren das Messen des Volumenanteils des Wassers und des Volumenanteils des mindestens einen Kohlenwasserstoffs in der periodischen und vorübergehenden Freisetzung des Fluids, das das Kondensat (19) mit sich führt, in der Nähe des vorderen Rands desselben aufweist, indem eine Störung des elektrischen Feldes verwendet wird, die auf der Zeitbereichsreflektometrie basiert.

**2.** Verfahren nach Anspruch 1, welches des Weiteren folgendes aufweist:

Bereitstellen einer länglichen Leitung (20) mit einer inneren Bohrung (28), die eine vorbestimmte, im wesentlichen gleichmäßige Innendurchmesserabmessung aufweist;
Bereitstellen von zwei Sonden (30A, 30B) zur Störung des elektrischen Feldes und Positionieren jeder der Sonden (30A, 30B) zur Störung des elektrischen Feldes zumindest teilweise innerhalb der Bohrung (28) der länglichen Leitung (20) und in einem bekannten und vorbestimmten Abstand relativ zueinander;
Bereitstellen eines Fluiddrucksensors, der in einer den Fluiddruck erfassenden Beziehung relativ zu der Innenbohrung (28) der länglichen Leitung (20) angebracht ist und der des Weiteren ein Fluiddrucksignal erzeugt;
Bereitstellen eines Temperatursensors (100), der in einer die Temperatur erfassenden Beziehung relativ zu der Innenbohrung (28) der länglichen Leitung (20) angebracht ist und der des Weiteren ein Fluidtemperatursignal erzeugt;
elektrisches Koppeln der beiden Sonden (30A, 30B) zur Störung des elektrischen Feldes, des Fluiddrucksensors und des Temperatursensors (100) in einer ein Signal übertragenden Beziehung relativ zu dem Computer (170);
Zuführen der Fluids (13), das den Hauptvolumen-Bestandteil und das mindestens eine Kondensat (19) enthält, in die Innenbohrung (28) der länglichen Leitung (20);
elektrisches Abtasten, mit dem Computer (170), jedes der jeweiligen zwei Sonden (30A, 30B) zur Störung des elektrischen Feldes, Fluiddruck und/oder Temperatursensoren; und
Korrelieren der von den mindestens Sonden (30A, 30B) zur Störung des elektrischen Feldes, dem Drucksensor und/oder dem Temperatursensor (100) empfangenen Signale mit dem Computer (170), um eine Charakterisierung des Fluids (13) zu liefern.

**3.** Verfahren nach Anspruch 2, wobei jeder der Sensoren zur Störung des elektrischen Feldes an vorbestimmten, voneinander beabstandeten Messstationen angeordnet ist, die entlang der länglichen Leitung (20) angeordnet sind; und
elektrisches Abtasten mit dem Computer (170) an jeder der Abtaststationen mit einer Geschwindigkeit von etwa 500 Bildern pro Sekunde.

**4.** Verfahren nach Anspruch 3, welches des Weiteren folgendes aufweist:
Berechnen einer Strömungsgeschwindigkeit des Fluids (13) durch die innere Bohrung (28) der länglichen Leitung (20) mit dem Computer (170) aus den von den beiden Sonden (30A, 30B) zur Störung des elektrischen Feldes und den Temperatur- und Fluiddrucksensoren empfangenen Signalen.

**5.** Verfahren nach Anspruch 4, welches des Weiteren folgendes aufweist:

Charakterisieren der durchschnittlichen Zusammensetzung des Fluids (13) in der Region der jeweiligen, beabstandeten Messstationen mit dem Computer (170) unter Verwendung der von den jeweiligen Sensoren zur Störung des elektrischen Feldes empfangenen Signale; und
Schätzen eines Gesamtdurchflussvolumens des Fluids (13) und eines Teilvolumens des mindestens einen Kohlenwasserstoffs, der mit dem Fluid (13) mitgeführt wird, unter Verwendung des Computers (170).

**6.** Verfahren nach Anspruch 5, welches des Weiteren folgendes aufweist:

Berechnen einer ungefähren Fluiddichte des Fluids (13) mit dem Computer (170) unter Verwendung der von den Temperatur- und Drucksensoren empfangenen Signale während einer gegebenen Abtastzeit; und

Bereitstellen einer zusätzlichen und extern montierten Vorrichtung (90A) zur Messung des Fluiddurchflusses und Koppeln der zusätzlichen und extern montierten Vorrichtung (90A) zur Messung des Fluiddurchflusses in einer Signalübertragungsbeziehung relativ zu dem Computer (170);

Zuführen des Fluids (13) zu der zusätzlichen und extern montierten Vorrichtung (90A) zur Messung des Fluiddurchflusses und Erzeugen eines Signals mit der zusätzlichen und extern montierten Vorrichtung (90A) zur Messung des Fluiddurchflusses, das an den Computer (170) übertragen wird;

Messen der Fluidströmungsbewegung der Quelle des Fluids (13) durch die zusätzliche und extern montierte Vorrichtung (90A) zur Messung des Fluiddurchflusses;

Schätzen des Gesamtdurchflusses des Fluids (13) mit dem Computer (170) unter Verwendung des von der zusätzlichen und extern montierten Vorrichtung (90A) zur Messung des Fluiddurchflusses erzeugten Signals; und

Verbessern der geschätzten Berechnung des Gesamtdurchflussvolumens des Fluids (13) und des Teilvolumens des mindestens einen Kohlenwasserstoffs, der mit dem Fluid (13) mitgeführt wird, unter Verwendung des geschätzten Gesamtdurchflusses des Fluids (13), der von der zusätzlichen und extern montierten Vorrichtung (90A) zur Messung des Fluiddurchflusses erfasst wird, unter Verwendung des Computers (170).

**7.** Verfahren nach Anspruch 1, welches des Weiteren folgendes aufweist:

Anwenden einer Fourier-Transformationsberechnung auf die erfasste elektrische Impulsreflexion, die von der Sonde (30, 30A, 30B) empfangen wird, und die verwendet wird, um eine Resonanzfrequenz und einen Resonanzpunkt von mindestens einem der Volumenbestandteile zu bestimmen.

**8.** Verfahren nach Anspruch 7, welches des Weiteren folgendes aufweist:

Anwenden einer Berechnung der Leistungsspektraldichte (PSD) mittels des Computers (170) auf die Fourier-Transformationsfrequenz (FT), um eine Amplitude und Stärke von mindestens einem der gegebenen Resonanzpunkte zu bestimmen.

**9.** Verfahren nach Anspruch 8, wobei der Volumenbestandteil eine Vielzahl von Volumenbestandteilen ist.

**10.** Verfahren nach Anspruch 9, und wobei die Vielzahl von Volumenbestandteilen eine Flüssigkeit und ein Gas aufweist.

**Revendications**

**1.** Procédé pour identifier et caractériser un rejet périodique d'un condensat donné (19) qui est entraîné dans une source d'un fluide (13), comprenant :

la fourniture d'une source d'un fluide (13) ayant une composition donnée qui comprend un constituant de fraction volumique majeure, et dans lequel au moins un condensat (19) est périodiquement libéré, puis est entraîné dans la source du fluide (13), et dans lequel le constituant de fraction volumique majeure, et ledit au moins un condensat (19) ont chacun une constante diélectrique préalablement déterminée et connue, et un point de résonance préalablement déterminé et connu ;

la fourniture d'une base de données (172) contenant des informations accessibles et stockées sur les constantes diélectriques préalablement déterminées et connues du constituant de fraction volumique majeure, et/ou dudit au moins un condensat (19), et des informations accessibles et stockées sur les points de résonance préalablement déterminés et connus de concentrations données du constituant de fraction volumique majeure, et/ou dudit au moins un condensat (19) ;

la fourniture d'une sonde (30, 30A, 30B) qui est exposée, au moins en partie, à la source de fluide (13), et qui a en outre une dimension de longueur connue ;

la fourniture d'un émetteur d'impulsions électriques (120) qui, lorsqu'il est sous tension, génère une impulsion électrique donnée qui est électriquement délivrée à la sonde (30, 30A, 30B), et dans lequel l'impulsion électrique se déplace électriquement le long de la dimension de longueur connue de la sonde (30, 30A, 30B), et génère en outre une réflexion d'impulsion électrique ;

la fourniture d'un dispositif d'échantillonnage d'impulsions électriques (150) qui est couplé électriquement dans la réception d'impulsions électriques et la relation de détection par rapport à la sonde (30, 30A, 30B) ;

la fourniture d'un ordinateur (170) qui est couplé électriquement à la sonde (30, 30A, 30B), à l'émetteur d'impulsions électriques (120), au dispositif d'échantillonnage d'impulsions électriques (150) et à la base de données (172), et dans lequel l'ordinateur (170) détermine une période de temps qui s'écoule entre l'émission de l'impulsion électrique envoyées dans la sonde (30, 30A, 30B) et la réception de la réflexion de l'impulsion électrique détectée reçue de la sonde (30, 30A, 30B), et dans lequel le point de résonance du constituant de fraction volumique majeure, et le point de résonance dudit au moins un condensat (19) sont calculés individuellement par l'ordinateur (170) à partir des périodes de temps déterminées, et/ou l'ordinateur (170) établit une corrélation entre la période de temps déterminée et les constantes diélectriques connues et déterminées précédemment, et dans lequel l'ordinateur (170) met ensuite en corrélation les points de résonance calculés du constituant de la fraction volumique majeure et dudit au moins un condensat (19), tels que fournis dans la base de données (172), de manière à identifier une caractéristique du constituant de la fraction volumique majeure et dudit au moins un condensat (19) qui est entraîné dans la source de fluide (13) ;

la fourniture d'une interface utilisateur qui est couplée électroniquement à l'ordinateur et qui génère en outre une sortie perceptible par l'utilisateur qui identifie respectivement ladite au moins une caractéristique du constituant de la fraction volumique majeure et ledit au moins un condensat ;

dans lequel le condensat (19) qui est entraîné dans la source de fluide (13) est libéré de manière transitoire et périodique, et dans lequel le procédé comprend en outre l'étape consistant à mesurer la libération transitoire et périodique du fluide et du condensat (19) sur une période de temps donnée ;

dans lequel la libération transitoire et périodique du condensat (19), et qui est en outre entraîné avec la source de fluide (13), a lieu sur une période de temps inférieure à environ 200 secondes ;

le procédé étant **caractérisé en ce qu'**il comprend en outre l'échantillonnage électrique de la source du fluide (13) ayant la composition donnée, et qui comprend le constituant de la fraction volumique majeure, et ledit au moins un condensat (19), pendant une période de temps pendant laquelle le constituant de la fraction volumique majeure, qui comprend ledit au moins un condensat (19), a la plus faible concentration d'une source de méthane gazeux ; et

dans lequel la libération périodique et transitoire du fluide entraînant le condensat (19) présente un bord avant, et contient en outre de l'eau, et au moins un hydrocarbure, chacun ayant un point de résonance prédéterminé et connu et une constante diélectrique prédéterminée et connue, et dans lequel le procédé comprend en outre la mesure de la fraction volumique de l'eau, et de la fraction volumique dudit au moins un hydrocarbure dans la libération périodique et transitoire du fluide entraînant le condensat (19) à proximité de son bord d'attaque, en utilisant une perturbation de champ électrique basée sur la réflectométrie dans le domaine temporel.

2. Procédé selon la revendication 1, comprenant en outre :

la fourniture d'un conduit allongé (20) ayant un alésage interne (28) qui a une dimension diamétrale intérieure prédéterminée, substantiellement uniforme ;
la fourniture de deux sondes de perturbation du champ électrique (30A, 30B), et le positionnement de chacune des sondes de perturbation du champ électrique (30A, 30B), au moins en partie, à l'intérieur de l'alésage (28) du conduit allongé (20), et à une distance connue et prédéterminée, l'une par rapport à l'autre ;
la fourniture d'un capteur de pression de fluide qui est monté dans une relation de détection de pression de fluide par rapport à l'alésage interne (28) du conduit allongé (20), et qui génère en outre un signal de pression de fluide ;
la fourniture d'un capteur de température (100) qui est monté en relation de détection de température par rapport à l'alésage interne (28) du conduit allongé (20), et qui génère en outre un signal de température de fluide ;
le couplage électrique des deux sondes de perturbation du champ électrique (30A, 30B), du capteur de pression de fluide et du capteur de température (100) dans une relation de transmission de signal par rapport à l'ordinateur (170) ;
la délivrance de la source de fluide (13) qui comprend le constituant de fraction volumique majeure, et dudit au moins un condensat (19), dans l'alésage interne (28) du conduit allongé (20) ;
l'échantillonnage électrique, à l'aide de l'ordinateur (170), des signaux de chacune des deux sondes de perturbation du champ électrique respectives (30A, 30B), de la pression du fluide, et/ou des signaux du capteur de température ; et
la corrélation des signaux reçus desdites au moins deux sondes de perturbation du champ électrique (30A,30B), du capteur de pression et/ou du capteur de température (100), avec l'ordinateur (170), de manière à fournir une caractérisation de la source du fluide (13).

3. Procédé selon la revendication 2, et dans lequel chacun des capteurs de perturbations électriques est situé à des stations de détection prédéterminées et espacées qui sont situées le long du conduit allongé (20) ; et

l'échantillonnage électrique avec l'ordinateur (170), à chacune des stations de détection, à une vitesse d'environ 500 images par seconde.

4. Procédé selon la revendication 3, et comprenant en outre :

le calcul, à l'aide de l'ordinateur (170), d'une vitesse d'écoulement de la source de fluide (13) à travers l'alésage interne (28) du conduit allongé (20) à partir des signaux reçus des deux sondes de perturbation du champ électrique (30A, 30B) et des capteurs de température et de pression du fluide.

5. Procédé selon la revendication 4, et comprenant en outre :

la caractérisation, à l'aide de l'ordinateur (170), de la composition moyenne de la source du fluide (13) dans la région des stations de détection espacées respectives, en utilisant les signaux reçus des capteurs de perturbation du champ électrique respectifs ; et

l'estimation, en utilisant l'ordinateur (170), d'un volume d'écoulement total de la source de fluide (13), et d'un volume fractionnaire dudit au moins un hydrocarbure qui est entraîné avec la source de fluide (13).

6. Procédé selon la revendication 5, et comprenant en outre :

le calcul, avec l'ordinateur (170), d'une densité approximative du fluide de la source du fluide (13), en utilisant les signaux reçus des capteurs de température et de pression, pendant un temps d'échantillonnage donné ; et

la fourniture d'un dispositif de mesure de débit de fluide auxiliaire et monté à l'extérieur (90A) et le couplage du dispositif de mesure de débit de fluide auxiliaire et monté à l'extérieur (90A) dans une relation de transmission de signal par rapport à l'ordinateur (170) ;

l'envoi de la source de fluide (13) au dispositif de mesure de débit de fluide auxiliaire et monté à l'extérieur (90A), et la génération d'un signal avec le dispositif de mesure de débit de fluide auxiliaire et monté à l'extérieur (90A) qui est transmis à l'ordinateur (170) ;

la mesure du mouvement de l'écoulement du fluide de la source de fluide (13) à travers le dispositif de mesure de débit de fluide auxiliaire et monté à l'extérieur (90A) ;

l'estimation du débit total de la source de fluide (13), avec l'ordinateur (170), en utilisant le signal généré par le dispositif de mesure de débit de fluide auxiliaire et monté à l'extérieur (90A) ; et l'amélioration du calcul estimé du volume d'écoulement total de la source de fluide (13), et du volume fractionnaire dudit au moins un hydrocarbure qui est entraîné avec la source de fluide (13), en utilisant le débit total estimé de la source de fluide (13), et qui est détecté par le dispositif de mesure de débit de fluide auxiliaire et monté à l'extérieur (90A), en utilisant l'ordinateur (170).

7. Procédé selon la revendication 1, et comprenant en outre :

l'application d'un calcul de transformée de Fourier à la réflexion d'impulsion électrique détectée reçue de la sonde (30, 30A, 30B), et qui est utilisé pour déterminer une fréquence de résonance et un point de résonance d'au moins un des constituants de la fraction volumique.

8. Procédé selon la revendication 7, et comprenant en outre :

l'application d'un calcul de densité spectrale de puissance (PSD), au moyen de l'ordinateur (170), à la fréquence de la transformée de Fourier (FT) de manière à déterminer une amplitude et une intensité d'au moins un des points de résonance donnés.

9. Procédé selon la revendication 8, et dans lequel le constituant de fraction volumique est une multiplicité de constituants de fraction volumique.

10. Procédé selon la revendication 9, et dans lequel la multiplicité de constituants de fraction volumique comprend un liquide et un gaz.

FIG. 1

FIG. 2

FIG. 3

FIG. 4

FIG. 5

FIG. 6

FIG. 6A

75

74

73

69

41

32

51

72

72

40

50

72

71

70

72

67

37

60

38

55

55

42

52

61

34

35

33

FIG. 7

FIG. 8

FIG. 9

FIG. 10

FIG. 11

FIG. 12

TDR Trace of Probe in Mineral Oil

FIG. 13

FIG. 14

FIG. 15

FIG. 16

PSD of Air, Oil and Water

FIG. 17

FIG. 18

EP 3 850 349 B1

FIG. 19

EP 3 850 349 B1

## PSD Water (reduced scale)

FIG. 20

FIG. 21

EP 3 850 349 B1

FIG. 22

FIG. 23

FIG. 24

FIG. 25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017350843 A1
- US 4786857 A
- US 5723979 A
- US 6144211 A
- US 6348803 B